# FASCICULE DE BREVET EUROPEEN

(11) **EP 2 828 279 B1**
(45) Date de publication et mention de la délivrance du brevet: **16.03.2016**
(21) Numéro de dépôt: 13713375.7
(22) Date de dépôt: 18.03.2013
(51) Int. Cl.: C07K 1/02, C07K 7/06, C07K 7/08, C07D 293/02, C07K 1/04

(54) **PROCÉDÉ DE LIGATION NATIVE**
NATIVES LIGATIONSVERFAHREN
NATIVE LIGATION METHOD

(30) Priorité: 21.03.2012 FR 1252548
(43) Date de publication de la demande: 28.01.2015
(73) Titulaire: Centre National de la Recherche Scientifique (CNRS), 75794 Paris Cedex 16 (FR); Institut Pasteur de Lille, 59019 Lille Cedex (FR); Université de Lille 2 Droit et Santé, 59800 Lille (FR)
(72) Inventeur: MELNYK, Oleg, F-59112 Annoeullin (FR); RAIBAUT, Laurent, F-06000 Nice (FR); OLLIVIER, Nathalie, F-59100 Roubaix (FR)
(74) Mandataire: Hirsch & Associés
(86) Numéro de dépôt international: PCT/EP2013/055507
(87) Numéro de publication internationale: WO 2013/139719

(56) Documents cités:
- WO-A1-2011/051906
- MARKUS MUTTENTHALER ET AL: "Selenopeptide chemistry", JOURNAL OF PEPTIDE SCIENCE, vol. 14, no. 12, 1 décembre 2008 (2008-12-01), pages 1223-1239, XP055044339, ISSN: 1075-2617, DOI: 10.1002/psc.1075

## Description

### DOMAINE DE L'INVENTION

La présente invention concerne un procédé de fabrication d'un polypeptide comprenant au moins une étape de ligation native à l'aide d'un peptide fonctionnalisé par un groupement sélénié. L'invention concerne également des peptides et des composés à base de sélénium. L'invention concerne également un kit de synthèse pour la synthèse de peptides.

### ARRIERE-PLAN TECHNOLOGIQUE

La synthèse de polypeptides par des méthodes conventionnelles en phase solide, acide aminé par acide aminé, est limitée par des rendements faibles lorsque les polypeptides synthétisés sont de grande taille. Afin de surmonter cette limitation, il est connu d'assembler deux polypeptides par ligation chimique afin de produire un polypeptide plus long.

La synthèse totale de polypeptides est de plus en plus utile pour la préparation de protéines de structures bien définies ou portant des modifications naturelles, comme les modifications post-traductionnelles, ou non naturelles. Les méthodes de ligation chimique apportent une réponse à ce besoin, cependant elles s'avèrent limitées dans leur emploi et leur application industrielle.

De manière générale, dans ces méthodes, on souhaite que le lien entre les polypeptides assemblés par ligation soit natif, c'est-à-dire corresponde à la structure naturelle des polypeptides.

La principale méthode de ligation native existant à ce jour est celle de Kent et Dawson, décrite par exemple dans les demandes internationales WO 96/34878 et WO 98/28434. Cette méthode est fondée sur une réaction chimiosélective entre un peptide thioester (en C terminal) et un cystéinyl-peptide. Néanmoins, le principal inconvénient de cette méthode est la fabrication des peptides thioesters qui nécessite des procédés chimiques complexes. Ces méthodes de l'art antérieur ne sont pas satisfaisantes car elles conduisent inévitablement à des mélanges pouvant être difficilement séparables, donc affectant la pureté du produit final obtenu, et à des pertes inévitables de rendement.

Une méthode alternative est la ligation dite de Staudinger, décrite dans les demandes internationales WO 01/68565 et WO 01/87920. Celle-ci comprend la réaction d'un phosphinothioester avec un azoture et l'hydrolyse des réactifs combinés pour former une liaison amide. Cependant cette méthode est difficilement applicable à l'échelle industrielle.

Une autre méthode, décrite dans la demande internationale WO 2007/037812, repose sur la réaction d'un α-cétoacide avec une N-alkoxyamine dans une réaction de condensation décarboxylative. Toutefois, les cétoacides sont des molécules qui sont difficiles à fabriquer et à incorporer dans des peptides. Aussi, cette troisième méthode est difficilement applicable dans les laboratoires de synthèse peptidique ne disposant pas des moyens de réaliser des synthèses organiques complexes.

Le document WO 2011/051906 décrit un procédé de ligation native faisant intervenir un composé thiol. Bien que cette méthode soit extrêmement satisfaisante, la cinétique de réaction de ce composé thiol est parfois lente, notamment lorsque les acides aminés encadrant la liaison entre les deux peptides sont encombrés.

Il subsistait donc le besoin d'une méthode de ligation native dotée d'une cinétique de réaction rapide de façon à rendre plus facile la ligation de peptides par formation d'une liaison entre deux acides aminés encombrés.

### RESUME DE L'INVENTION

L'invention repose sur la mise au point d'une méthode de ligation basée sur l'utilisation d'un peptide amide particulier, le peptide SeEA, et d'un cystéinyl peptide. Lorsque ces deux peptides sont mis en contact en solution aqueuse, un lien peptidique natif se forme entre les deux fragments. La ligation est très efficace et s'effectue avec d'excellents rendements. Elle fonctionne dans une large gamme de pH.

Cette ligation présente des similitudes avec la ligation SEA, décrite dans la demande FR 09 57 639, bien que les propriétés de ces deux ligations et des segments SeEA et SEA soient significativement différentes.

En effet, les cinétiques de ligation SeEA sont *très* significativement supérieures aux cinétiques de ligation SEA, obtenues avec l'analogue souffré SEA, ou aux cinétiques de la Native Chemical Ligation (NCL) basée sur l'utilisation de peptides thioesters.

La supériorité cinétique de SeEA ne diminue en rien l'intérêt de la ligation SEA. Au contraire, elle vient la compléter en permettant la formation de jonctions difficiles à produire par les autres techniques de ligation SEA ou NCL (chimie des thioesters).

Dans la présente demande, le groupement SEA représente un groupement -N(CH₂-CH₂-S-G₄)₂, avec G₄ représentant H ou un groupement protecteur du soufre ou une liaison disulfure.

Un premier objet de la présente invention concerne un peptide fonctionnalisé choisi parmi :
a) le peptide de formule (I) : X₁-N(CH₂CH₂SeH)₂ ; ou
b) le peptide de formule (I') : 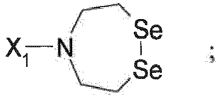 dans lesquels X₁ représente un fragment peptidique et le groupement -N(CH₂CH₂SeH)₂ ou 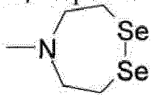 forme une liaison amide avec la terminaison C=O du résidu d'acide aminé du fragment peptidique X₁ qui est en position C-terminale.
Selon un mode de réalisation, X₁ comprend de 2 à 300 résidus d'acides aminés, de préférence de 5 à 100 résidus d'acides aminés, de manière plus particulièrement préférée de 8 à 50 résidus d'acides aminés.

Un autre objet de la présente invention concerne un procédé d'obtention d'un peptide fonctionnalisé (I) ou (I') selon l'invention, comprenant les étapes a1 et b1 suivantes :
a1) synthèse peptidique pour fournir un polypeptide de formule (II) :

   (II) X₁-OH

   comportant de préférence des groupements protecteurs sur ses fonctions amines et acides carboxyliques, à l'exception de sa fonction acide carboxylique C-terminale,
b₁) fonctionnalisation du polypeptide obtenu à l'étape a) comprenant :
   - la réaction du polypeptide de formule (II) avec un composé amine choisi parmi :

      (IIIa) NH(CH₂-CH₂-Se-G₁)₂

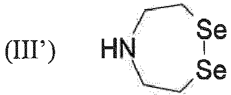

      dans laquelle G₁ représente un groupement protecteur du sélénium ou un atome d'hydrogène,
      en phase liquide, pour former le polypeptide de formule (Ia) X₁-N(CH₂CH₂-Se-G₁)₂, ou (I') ;
   - éventuellement la déprotection du polypeptide de formule (Ia) pour conduire au composé de formule (I),
ou comprenant les étapes a2 et b2 suivantes :
a2) l'utilisation
   - du composé de formule (IV) ou (IV") :

      (IV) X₁-N(CH₂CH₂-S-H)₂ ;

      Ou 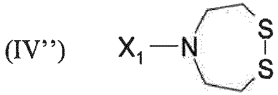
   - d'un peptide fonctionnalisé par un groupement porteur d'une fonction thioester (IV') = X₁ -SR et R représente un groupement alkyle ou aryle éventuellement substitué,
b2) la réaction entre le composé de formule (IV) ou (IV") ou le composé de formule (IV') avec le composé amine de formule (III') ou (III") : 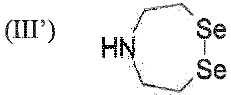 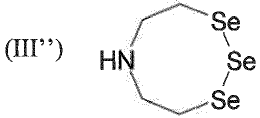 en phase liquide, pour former le peptide de formule (I) ou (I').

Un autre objet de la présente invention concerne un composé susceptible d'être utilisé dans le procédé d'obtention d'un peptide fonctionnalisé selon l'invention, ce composé étant choisi parmi :
a) le composé de formule (III') : 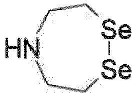
b) le composé de formule (III") :

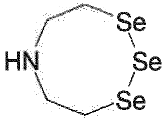

L'invention concerne également un procédé de fabrication des composés de formules (III') et (III") selon l'invention, comprenant la réaction suivante de traitement par un hydrure métallique illustrée sur le schéma dans le cas de NaBH₄, mais on peut également employer LiAlH₄ ou LiBH₄ : 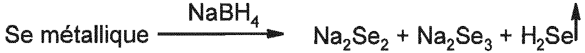 suivie de la réaction :

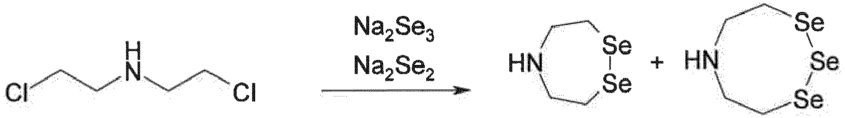

Un autre objet de la présente invention concerne un procédé d'obtention d'un peptide fonctionnalisé (I) ou (I') selon l'invention, comprenant les étapes suivantes :
a) Fourniture d'une résine polymère fonctionnalisée présentant la structure (V) : 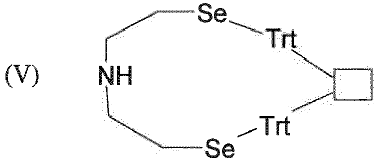 Ou la structure (V') : 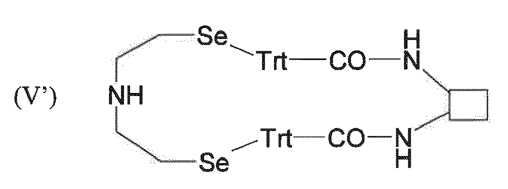 dans lesquelles,
   □ représente un support solide,
   Trt représente un groupement triphénylméthyle éventuellement substitué par un ou plusieurs substituants, notamment choisi parmi les substituants chlore, méthoxy, méthyle, fluor et cyano.
b) Greffage d'un acide aminé sur la résine polymère fonctionnalisée obtenue à l'étape a) pour conduire au composé de structure (VI) : 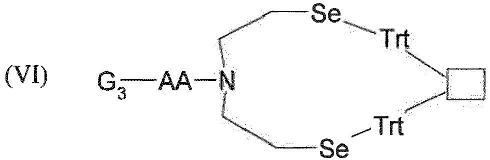 Ou de structure (VI') : 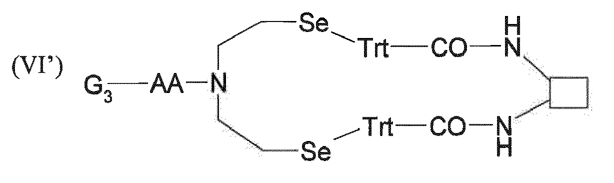 dans lesquelles,
   □ et Trt ont la même signification que dans le composé (V) ou (V'),
   AA représente un résidu d'acide aminé comportant éventuellement un ou plusieurs groupements protecteurs ;
   G₃ représente un atome d'hydrogène ou un groupement protecteur de la fonction amine N-terminale de AA,
c) Synthèse peptidique à partir de l'acide aminé AA pour conduire au composé (VII) ou (VII') : 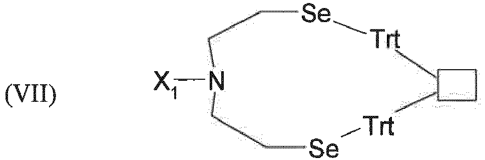 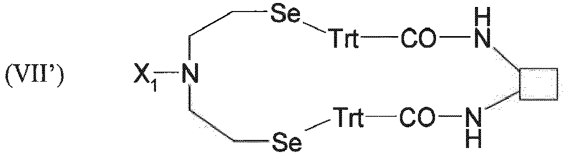 dans lesquelles,
   □ représente un support solide,
   Trt représente un groupement triphénylméthyle éventuellement substitué par un ou plusieurs substituants, notamment choisi parmi les substituants chlore, méthoxy, méthyle, fluor et cyano,
   X₁ représente un fragment peptidique,
d) Coupure de la liaison entre Trt et Se pour conduire au composé (I) ou (I').

Selon un mode de réalisation du procédé ci-dessus, le greffage d'un acide aminé sur la résine polymère comprend la mise en présence de la résine fonctionnalisée (V) ou (V') avec un halogénure d'acide aminé ou avec un acide aminé et un agent d'activation, choisi de préférence parmi l'HATU, le PyBOP, le BOP, le PyBROP, de manière plus particulièrement préférée l'HATU.

Un autre objet de la présente invention concerne une résine polymère fonctionnalisée susceptible d'être utilisée dans le procédé selon l'invention, présentant la structure (V) :

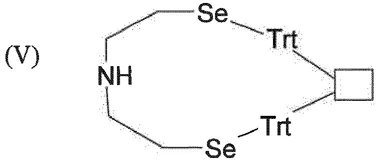

Ou la structure (V') : 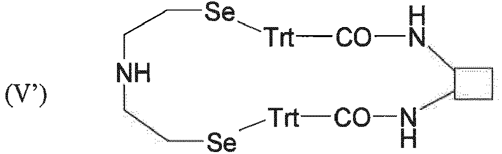 dans lesquelles,
□ représente un support solide,
Trt représente un groupement triphénylméthyle éventuellement substitué par un ou plusieurs substituants, notamment choisi parmi les substituants chlore, méthoxy, méthyle, fluor et cyano.

Un autre objet de la présente invention concerne une résine polymère comprenant un fragment peptidique répondant à la formule (VII) ou (VII') : 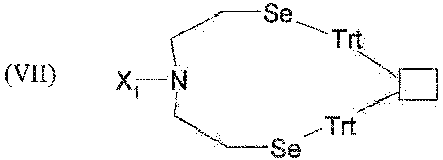 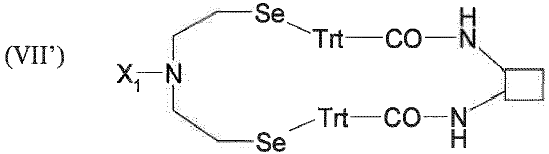 dans lesquelles,
D représente un support solide,
Trt représente un groupement triphénylméthyle éventuellement substitué par un ou plusieurs substituants, notamment choisi parmi les substituants chlore, méthoxy, méthyle, fluor et cyano,
X₁ représente un fragment peptidique.

Selon un mode de réalisation, le support solide □ est choisi parmi un polystyrène, un polyacrylamide, un polyéthylèneglycol, une cellulose, un polyéthylène, un polyester, un latex, un polyamide, un polydiméthylacrylamide, un copolymère polyéthylèneglycol-polystyrène, un copolymère polyéthylèneglycol-polyacrylamide et leurs dérivés.

Un autre objet de la présente invention concerne un procédé de fabrication de la résine polymère selon l'invention, comprenant :
a) la fourniture d'un support solide fonctionnalisé par des groupements -Trt ou -NH-CO-Trt ;
b) réaction de la fonction Trt du support solide avec le composé amine de formule (III) : NH(CH₂-CH₂-Se-H)₂ Trt ayant la même définition que ci-dessus.

Un autre objet de la présente invention concerne un procédé de fabrication d'un polypeptide de formule (VIII) :

(VIII) X₁-C₁-X₂-C₂-...-Cᵢ₋₁-Xᵢ-...-Cₙ₋₁-Xₙ

dans lequel,
X₁, X₂,..., Xᵢ,..., Xₙ sont des fragments peptidiques,
C₁, C₂, ..., Cᵢ₋₁, ..., Cₙ₋₁ sont des résidus d'acides aminés portant une fonction thiol ou sélénol,
n est un entier supérieur ou égal à 2,
i est un nombre entier quelconque allant de 1 à n, ce procédé comprenant une ou plusieurs étapes de réaction entre un composé de formule (IXᵢ) :

   (IXᵢ) X'ᵢ - N(CH₂CH₂-Se-H)₂

   dans laquelle,
   X'₁ représente X₁
   X'ᵢ représente Cᵢ₋₁-Xᵢ pour i > 1
   et un composé de formule (Xᵢ₊₁) :

   (Xᵢ₊₁) Cᵢ-Xᵢ₊₁-...-Cₙ₋₁-Xₙ

   pour former un composé de formule (Xᵢ) :

   (Xᵢ) X'ᵢ-Cᵢ-Xᵢ₊₁-...-Cₙ₋₁-Xₙ

Selon un mode de réalisation, le composé (IXᵢ) est formé à partir du composé (IX'ᵢ) : 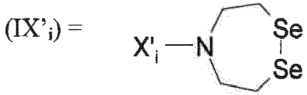 par la mise en présence d'au moins un composé réducteur des liaisons disélénium.

Selon un mode de réalisation, le composé (IX'ᵢ) est obtenu par réaction entre un composé de formule (XIᵢ), (XI'ᵢ) ou (XI"ᵢ) :

(XIᵢ) X'ᵢ-S-R₁

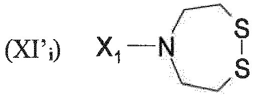

(XI"ᵢ) X'ᵢ-N(CH₂-CH₂-S-H)₂

dans lesquelles X'ᵢ est un fragment peptidique de la forme Cᵢ₋₁-Xᵢ pour i > 1 et X'₁ représente X₁, R₁ représente un groupement alkyle ou aryle, éventuellement substitué,
et un composé de formule (III') ou de formule (III") selon l'invention.

Selon un mode de réalisation, le procédé ci-dessus est mis en oeuvre pour la fabrication d'un polypeptide représenté par la formule (XII) :

(XII) X₁-C₁-X₂-C₂-X₃

comprenant les étapes suivantes :
a) réaction entre un composé de formule (XIII) ou (XIII') :

   (XIII) R₂-X₁-N(CH₂-CH₂-Se-H)₂

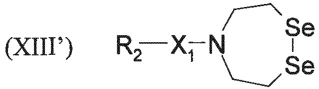

   et un composé de formule (XIV) ou (XIV') :

   (XIV) H-C₁-X₂-N(CH₂-CH₂-S-H)₂

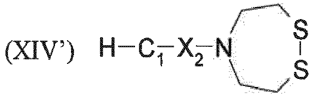

   pour former le composé de formule (XV) ou (XV') :

   (XV) R₂-X₁-C₁-X₂-N(CH₂-CH₂-S-H)₂ 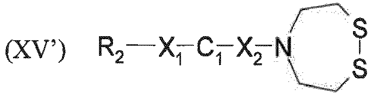
b) réaction entre un composé de formule (XV) ou (XV') et un polypeptide de formule (XVI) :

   (XVI) H-C₂-X₃

   pour former le composé de formule (XII),
   dans lesquelles, R₂ représente H ou un groupement protecteur de la fonction N-terminale de X₁ ; X₁, X₂, X₃ représentent, indépendamment les uns des autres, des fragments peptidiques ; C₁ et C₂ représentent, indépendamment l'un de l'autre, des résidus d'acides aminés porteurs d'une fonction thiol ou sélénol.

Un autre objet de la présente invention concerne un procédé de fabrication d'un polypeptide cyclique de formule (XVII) : 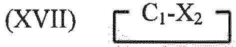 X₂ représentant un fragment peptidique, et C₁ représentant un résidu d'acide aminé comportant une fonction thiol ou sélénol,
ledit procédé comprenant au moins une étape de réaction de ligation d'un polypeptide de formule (XVIII) :

(XVIII) H-C₁-X₂-N(CH₂-CH₂-Se-H)₂

avec lui-même.

Selon un mode de réalisation du procédé de fabrication d'un polypeptide cyclique selon l'invention, la réaction de ligation est effectuée en mettant en contact un polypeptide de formule (XVIII') : 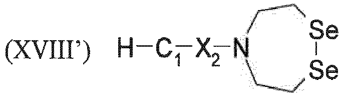 avec au moins un composé réducteur des liaisons disélénium.

Un autre objet de la présente invention concerne un kit de synthèse de polypeptides comprenant un ou plusieurs peptides de formules (IXᵢ) ou (IX'ᵢ) susceptibles d'être utilisés dans le procédé de ligation selon l'invention, ou au moins un composé (III') ou (III") selon l'invention.

Des avantages de la présente invention sont les suivants :
- la ligation native de l'invention permet d'assurer la liaison de jonctions difficiles, telles que les jonctions encombrées,
- la cinétique de ligation native de l'invention est très rapide,
- la ligation native de l'invention peut être combinée avec d'autres méthodes de ligation ayant des cinétiques différentes,
- la ligation native de l'invention est simple à mettre en oeuvre,
- la fabrication des peptides SeEA peut être faite à partir d'un support solide,
- La fabrication des peptides SeEA peut être faite à partir de segments SEA ou thioester en solution
- la ligation native de l'invention peut être mise en oeuvre à partir de fragments peptidiques qui peuvent être préparés en phase solide ou en phase liquide,
- la ligation native de l'invention peut être combinée avec d'autres types de ligations natives de l'art antérieur dans un procédé one-pot comportant plusieurs ligations successives,
- la ligation native de l'invention peut être effectuée en mettant en oeuvre un procédé de ligation de l'art antérieur par simple addition d'un composé sélénié particulier dans le mélange réactionnel.

### BREVE DESCRIPTION DES FIGURES

D'autres caractéristiques et avantages de l'invention apparaîtront à la lecture de la description qui suit d'un mode de réalisation préféré de l'invention, donnée à titre d'exemple et en référence au dessin annexé.
Les figures 1a et 1b représentent respectivement le chromatogramme HPLC et le spectre de masse d'un composé sélénié selon l'invention.
Les figures 2a et 2b représentent respectivement le chromatogramme HPLC et le spectre de masse d'un composé sélénié selon l'invention.
Les figures 3a et 3b représentent des chromatogrammes HPLC pour le suivi de la synthèse d'un peptide fonctionnalisé selon l'invention.
La figure 3c représente le spectre de masse d'un peptide fonctionnalisé selon l'invention.
Les figures 4a et 4b représentent respectivement le chromatogramme HPLC et le spectre de masse d'un peptide fonctionnalisé selon l'invention.
Les figures 5a et 5b représentent respectivement le chromatogramme HPLC et le spectre de masse d'un peptide fonctionnalisé selon l'invention après purification.
Les figures 6a et 6b représentent respectivement le chromatogramme HPLC et le spectre de masse d'un polypeptide obtenu par un procédé de ligation selon l'invention.
La figure 7 représente un diagramme mesurant la cinétique de réaction d'un procédé de ligation selon l'invention et selon l'art antérieur.
La figure 8 représente un diagramme mesurant la cinétique de réaction d'un procédé de ligation selon l'invention et selon l'art antérieur.
Les figures 9a et 9b représentent respectivement le chromatogramme HPLC et le spectre de masse d'un polypeptide fonctionnalisé obtenu par un procédé selon l'invention.
Les figures 10a et 10b représentent respectivement le chromatogramme et le spectre de masse d'un polypeptide obtenu par un procédé selon l'invention.
Les figures 11a et 11b représentent respectivement le chromatogramme et le spectre de masse d'un polypeptide obtenu par un procédé selon l'invention après purification.
La figure 12 représente un diagramme mesurant la cinétique de réaction d'un procédé de ligation selon l'invention et selon l'art antérieur.
La figure 13 représente un chromatogramme HPLC d'une solution issue de la synthèse d'un peptide fonctionnalisé selon l'invention.
Les figures 14a et 14b représentent respectivement le chromatogramme HPLC et le spectre de masse d'un peptide fonctionnalisé selon l'invention.

### DESCRIPTION DE MODES DE REALISATION DE L'INVENTION

L'invention est maintenant décrite plus en détail et de façon non limitative dans la description qui suit.

Par « peptide ou polypeptide » on entend, dans le cadre de la présente demande, une chaîne linéaire de résidus d'acides aminés (en nombre supérieur ou égal à deux) reliés par des liaisons peptidiques. Les « peptides ou polypeptides » au sens de la présente demande peuvent donc par exemple être des oligopeptides, peptides ou protéines selon l'acceptation conventionnelle de ces termes. Les résidus d'acides aminés présents dans les polypeptides selon l'invention peuvent être choisis parmi les résidus d'acides aminés protéinogéniques ou non. De préférence, ils sont choisis parmi les vingt résidus d'acides aminés protéinogéniques et la sélénocystéine.

La notation des polypeptides s'effectue de l'extrémité N-terminale vers l'extrémité C-terminale. Les résidus d'acides aminés présents le long de la chaîne polypeptidique sont notés selon le code usuel à une lettre ou à trois lettres. Un résidu d'acide aminé est un fragment de polypeptide de formule -NH-(CH-R)-(C=O)-, dans laquelle R représente une chaîne latérale, différente d'un acide aminé à l'autre.

Par « fragment peptidique » on entend, dans le cadre de la présente demande, une portion de polypeptide comprenant au moins un résidu d'acide aminé. Un fragment peptidique, au sens de la présente demande, peut donc être par exemple : une séquence de résidus d'acides aminés (telle que -AHG- ou -Ala-His-Gly-) si le fragment peptidique ne comprend ni l'extrémité N-terminale ni l'extrémité C-terminale du polypeptide ; ou bien une séquence de résidus d'acides aminés présentant un groupement à son extrémité N-terminale (telle que H-AHG- ou H-Ala-His-Gly-) si le fragment peptidique comprend l'extrémité N-terminale du polypeptide ; ou bien une séquence de résidus d'acides aminés présentant un groupement à son extrémité C-terminale (telle que -AHG-OH ou -Ala-His-Gly-OH) si le fragment peptidique comprend l'extrémité C-terminale du polypeptide.

### Peptide fonctionnalisé

Un premier objet de l'invention concerne un peptide fonctionnalisé par un groupement sélénié choisi parmi :
a) le peptide de formule (I): X₁-N(CH₂CH₂SeH)₂;
b) le peptide de formule (I') : 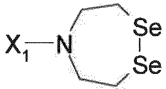 dans lesquels X₁ représente un fragment peptidique et le groupement -N(CH₂CH₂SeH)₂ ou 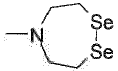 forme une liaison amide avec la terminaison C=O du résidu d'acide aminé du fragment peptidique X₁ qui est en position C-terminale.

Le fragment peptidique X₁ est de la forme Y₁-AA₁-AA₂-...-AAₙ dans laquelle Y₁ est un groupement N-terminal, de préférence un atome d'hydrogène, mais éventuellement aussi tout groupement substituant pour les amines primaires ou secondaires connu de l'homme du métier, par exemple un groupement acyle et notamment un groupement acétyle ; n est un nombre entier supérieur ou égal à 2 ; AA₁, AA₂, ..., AAₙ représentent, indépendamment les uns des autres, des résidus d'acides aminés.

Le polypeptide de formule (I) ou (I') comprend de préférence de 2 à 300 résidus d'acides aminés, de préférence de 5 à 100 résidus d'acides aminés, de manière plus particulièrement préférée de 8 à 50 résidus d'acides aminés.

Les polypeptides de formule (I) ou (I') comprennent de préférence uniquement des résidus d'acides aminés choisis parmi les vingt résidus d'acides aminés protéinogéniques et la sélénocystéine. Toutefois, selon un mode de réalisation particulier, les polypeptides de formule (I) ou (I') comprennent un ou plusieurs résidus d'acides aminés non-protéinogéniques.

Les résidus d'acides aminés des polypeptides de formule (I) ou (I') peuvent éventuellement être protégés par des groupements protecteurs des chaînes latérales.

### Composé amine de formule (III') et (III")

Un autre objet de l'invention concerne les composés amines de formule (III') et (III") ci-dessous et leur procédé de fabrication.

Les composés (III') et (III") répondent aux formules :

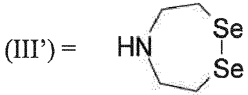

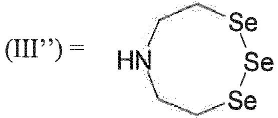

Les composés (III') et (III") peuvent être obtenus selon le procédé comprenant l'étape a) suivante : 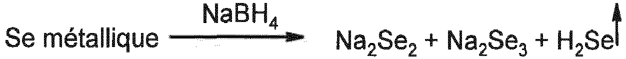 suivie de l'étape b) suivante :

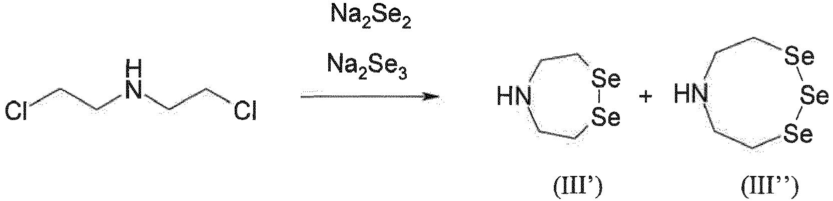

La première étape ci-dessus (étape a) est effectuée de préférence sous atmosphère d'azote. De préférence, les réactifs sont introduits à froid, puis, le mélange est porté à reflux.

Selon un autre mode de réalisation, dans l'étape a), NaBH₄ est remplacé par un autre hydrure métallique, tel que LiAlH₄ ou LiBH₄, Dans le cas des deux hydrures métalliques cités, les composés Li₂Se₂ et Li₂Se₃ sont formés. Ces deux composés Li₂Se₂ et Li₂Se₃ sont ensuite utilisés dans l'étape b) qui suit.

De préférence, la seconde étape (étape b) est réalisée à température ambiante.

De préférence, la phase organique comprenant les produits (III') et (III") est purifiée à l'issue du procédé, par exemple, par chromatographie liquide haute performance.

### Procédé d'obtention des composés (I) et (I') en phase liquide

Un autre objet de l'invention concerne un procédé d'obtention du polypeptide fonctionnalisé (I) ou (I') en phase liquide, ce procédé comprend une première étape de synthèse peptidique et une seconde étape de fonctionnalisation.

La première étape (étape de synthèse peptidique) permet d'obtenir le polypeptide de formule :

(II) X₁-OH

Cette étape de synthèse peptidique peut être effectuée selon toute méthode connue de l'homme du métier. Elle peut en particulier être effectuée en phase liquide ou, de préférence, en phase solide.

De manière schématique, la synthèse peptidique comprend une succession de couplages d'acides aminés à partir d'une amorce (acide aminé initial ou fragment peptidique résultant des additions d'acides aminés précédentes) et de déprotections. Plus précisément, la synthèse peptidique peut successivement comprendre :
(a) la fourniture d'un fragment peptidique présentant une extrémité N-terminale non protégée, et d'un acide aminé protégé à son extrémité N-terminale ;
(b) l'établissement d'une liaison peptidique entre l'acide aminé et le fragment peptidique, à l'extrémité N-terminale dudit fragment peptidique ;
(c) la déprotection de l'extrémité N-terminale de l'acide aminé lié, pour fournir le fragment peptidique de l'étape (a) suivante.

Au cours des différentes réactions de couplage, il est avantageux d'utiliser un composé activateur, notamment un carbodiimide (par exemple dicyclohexylcarbodiimide ou diisopropylcarbodiimide) en présence d'un triazole (par exemple 1-hydroxy-benzotriazole ou 1-hydroxy-7-azabenzotriazole), ou un sel phosphonium ou uronium d'un anion non-nucléophile (par exemple HBTU, HATU, TBTU ou PyBOP) ; ou encore d'utiliser des acides aminés activés sous forme d'halogénures d'acide, par exemple de fluorures d'acide.

Au cours des différentes réactions de couplage, les extrémités N-terminales des acides aminés sont avantageusement protégées par un groupement protecteur, de préférence un groupement Fmoc (9H-fluorèn-9-ylméthoxycarbonyle) ou encore un groupement t-Boc (tert-butoxycarbonyle) ou NSC (2-(4-nitrophénylsulfonyl)éthoxycarbonyle).

De même, les chaînes latérales des résidus d'acides aminés sont de préférence protégées au cours des différentes réactions de couplage par un ou plusieurs groupements protecteurs appropriés, par exemple un groupement tert-butyle pour les chaînes portant une fonction acide carboxylique.

Dans ce cas, une fois la dernière réaction de couplage d'acide aminé effectuée, on peut prévoir une réaction de déprotection des chaînes latérales. Il faut toutefois noter qu'il peut être préférable de conserver l'ensemble des protections (à l'exception d'une éventuelle protection de la fonction COOH à l'extrémité C-terminale) en vue de l'étape de fonctionnalisation.

### Fonctionnalisation

L'étape de fonctionnalisation comprend successivement :
- Eventuellement, la protection de l'extrémité N-terminale du polypeptide de formule (II) avec un groupement protecteur, de préférence choisi parmi la famille des carbamates, des amides ou des groupements alkyles, et notamment un groupement tert-butoxycarbonyle (t-Boc), Fmoc (9H-fluorèn-9-ylméthoxycarbonyle), trifluoroacétyle ou triphénylméthyle.
- Eventuellement également, la protection des fonctions des chaînes latérales des résidus d'acides aminés du polypeptide de formule (II), et tout particulièrement des fonctions amines (de préférence au moyen des groupements protecteurs ci-dessus) et des fonctions acides carboxyliques (au moyen par exemple de groupements tert-butyles).
- Alternativement, et selon un mode de réalisation plus simple, on peut fournir le polypeptide de formule (II) directement sous une forme dans laquelle l'ensemble des fonctions amines et acides carboxyliques sont protégées, en prévoyant une déprotection sélective de la fonction COOH de l'extrémité C-terminale.
- La réaction de couplage en phase liquide du polypeptide de formule (II) avec un composé amine choisi parmi les composés (IIIa) et (III') où :

   (IIIa) = NH(CH₂-CH₂-Se-G₁)₂

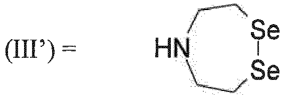

   dans lesquels G₁ représente un groupement protecteur du sélénium,
- Eventuellement la déprotection du polypeptide.

Selon un mode de réalisation, le groupement protecteur G₁ du composé (IIIa) est choisi parmi les groupements de type triphénylméthyle et benzyle et en particulier parmi le para-méthylbenzyle ou para-méthoxybenzyle.

Dans le cas où le composé (IIIa) est utilisé comme réactif de départ pour le couplage avec le composé (II), une étape ultérieure de déprotection du groupement G₁ est prévue afin d'obtenir le composé (I) ou (I'). De préférence, la déprotection s'effectue par traitement avec un acide comme l'acide trifluoroacétique ou l'acide fluorhydrique, ou un oxydant comme l'iode.

Un activateur est avantageusement présent lors de la réaction de couplage, par exemple l'hexafluorophosphate de benzotriazol-1-yl-oxytripyrrolidinophosphonium (PyBOP) ou l'hexafluorophosphate de bromo-trispyrrolidinophosphonium (PyBROP) ou l'acide aminé C-terminal lui-même est activé sous la forme d'un dérivé halogéné (en particulier sous la forme d'un fluorure d'acide aminé ou chlorure d'acide aminé), celui-ci pouvant être préformé ou formé in situ à l'aide de réactifs appropriés connus de l'homme de l'art. Parmi les halogénures d'acides aminés, les fluorures d'acides aminés sont préférés, préformés par réaction avec la 1,3,5-trifluorotriazine ou formés in situ à l'aide de TFFH (tétraméthylfluoroformamidinium hexafluorophosphate).

De manière générale, on peut également envisager tout réactif permettant l'activation de la fonction acide carboxylique de l'acide aminé connu de l'homme de l'art comme l'HBTU, le TBTU, l'HATU, le BOP... (on pourra se rapporter par exemple à Chemical approaches to the synthesis of peptides and proteins par Lloyd-Williams, P., Albericio, F., Giralt, E., 1997, CRC Press). Le PyBOP, le PyBROP, le BOP (l'hexafluorophosphate de benzotriazole-1-yl-oxy-tris-(dimethylamino)-phosphonium) ou de façon plus générale les phosphoniums sont préférés.

Le polypeptide fonctionnalisé (I) ou (I') selon l'invention peut être obtenu selon une seconde variante.

Les segments SeEA peuvent être formés séparément par échange à partir d'un segment SEA de type X₁-N(CH₂CH₂-S-G₄)₂ ou thioester X₁-S-R₁ à l'aide des réactifs de structure (III') ou (III"), G₄ représentant un atome d'hydrogène, un groupement protecteur des fonctions thiol ou une liaison disulfure et R₁ représentant un atome d'hydrogène ou un groupement alkyle ou aryle, éventuellement substitué..

Les segments SeEA peuvent également être formés *in situ,* pendant la ligation par échange à partir d'un segment SEA de type X₁-SEA ou thioester X₁-SR₁ à l'aide des réactifs (III') ou (III"). Le segment de type H-Cys-X₂ présent en solution consomme le segment X₁-SeEA formé par échange. En général, le segment X₁-SeEA formé in situ n'est pas observé puisqu'il réagit très rapidement avec le segment H-Cys-X₂. Du fait de la rapidité de l'échange et de la ligation SeEA, il en résulte une accélération très significative de la ligation, dès lors que le réactif (III') ou (III") est introduit dans le milieu réactionnel de ligation.

Ainsi, selon une seconde variante, l'obtention des composés (I) et (I') comprend les étapes successives suivantes :
a2) la fourniture
   - d'un peptide fonctionnalisé sur son extrémité C-terminale par un groupement porteur d'une fonction thiol répondant à la formule (IV) ou (IV") :

      (IV) = X₁-N(CH₂CH₂-S-H)₂ 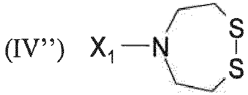
   - d'un peptide fonctionnalisé par un groupement porteur d'une fonction thioester (IV') = X₁ -SR et R représente un groupement alkyl ou aryl éventuellement substitué,
b2) la réaction en phase liquide entre le composé de formule (IV) ou le composé de formule (IV") ou le composé de formule (IV') et un composé amine de formule (III') ou (III") : 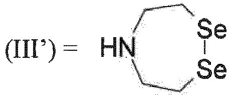 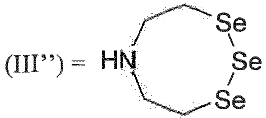 en présence éventuellement d'un composé réducteur des liaisons disulfures dans le cas où le composé (IV") est utilisé.

Le composé de formule (IV) ou de formule (IV") peut notamment être obtenu selon le protocole décrit dans le document WO 2011/051906.

Dans la formule (IV'), le groupement R représente de préférence un alkyl en C1-C12 éventuellement substitué ou un aryle en C6-C12 éventuellement substitué. De tels composés sont décrits notamment par WO96/34878 et WO98/28434.

Selon un mode de réalisation, le composé réducteur des liaisons disulfures peut être choisi parmi les composés thiol, tels que l'acide 4-mercaptophénylacétique (MPAA), le dithiothréitol (DTT), le thiophénol et ses dérivés, un alkylthiol (notamment le benzylmercaptan), parmi les composés sélénol comme le sélénophénol ou le diphénylsélénure ou parmi les phosphines telles que la tris(2-carboxyéthyl)phosphine (TCEP), ou leurs mélanges.

A l'issue de la réaction de couplage précédente, le composé (I') est obtenu. Le composé (I) est obtenu en mettant en contact le composé (I') avec un composé réducteur des liaisons disélénium ou trisélénium.

Parmi les composés connus comme réducteurs des liaisons di-sélénium ou trisélénium, on peut citer : la tris(2-carboxyéthyl)phosphine (TCEP), les thiols réducteurs comme l'acide 4-mercaptophénylacétique (MPAA), le thiophénol, le benzylmercaptan, le dithiothréitol (DTT), le sélénophénol ou le diphénylsélénure plus un agent réducteur des liaisons disélénium, le zinc métallique, les hydrures comme NaBH₄, LiBH₄, NaBH₃CN, NaBH(OAc)₃.

A l'issue de l'étape de fonctionnalisation, le polypeptide de formule (I) ou (I') est obtenu. Il est avantageux de prévoir à ce stade une étape de purification du composé, par exemple par chromatographie en phase liquide.

### Résine polymère fonctionnalisée

Un autre objet de l'invention concerne les résines polymères fonctionnalisées répondant à la structure (V) ou (V') : 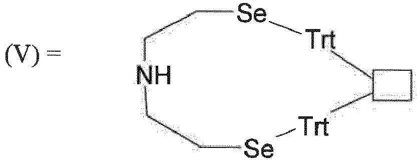 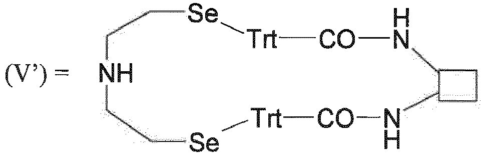 dans lesquelles,
□ représente un support solide,
Trt représente un groupement triphénylméthyle éventuellement substitué par un ou plusieurs substituants, notamment choisi parmi les substituants chlore, méthoxy, méthyle, fluor et cyano.

Ces résines polymères sont ensuite utilisées pour préparer le peptide fonctionnalisé SeEA.

A titre dé support solide D, on utilise un polymère soluble ou insoluble, le polymère insoluble étant de préférence sous forme de particules (billes). On peut par exemple utiliser une résine à base de polystyrène (de préférence) ou encore à base de polyacrylamide, polyéthylèneglycol, cellulose, polyéthylène, polyester, latex, polyamide, polydiméthylacrylamide, copolymère polyéthylèneglycol-polystyrène, copolymère polyéthylèneglycol-polyacrylamide ou encore une résine dérivée de celles-ci.

Pour préparer la résine polymère fonctionnalisée (V) ou (V'), on utilise un support solide □ porteur d'une fonctionnalisation adaptée, □-Z, dans laquelle Z est un groupement lieur. De préférence, le support solide présente des groupements chloro-triphénylméthyle (ou chlorotrityle), où le triphénylméthyle est éventuellement substitué par un ou plusieurs substituants, notamment choisis parmi les substituants chlore, méthoxy, méthyle, fluor et cyano.

A titre d'exemples de support solide □-Z fonctionnalisé, on peut citer les résines polystyrènes présentant des groupements lieurs chlorure de trityle, chlorure de 2-chlorotrityle, chlorure de 4-méthyltrityle ou chlorure de 4-méthoxytrityle. De tels supports solides sont disponibles commercialement, par exemple auprès de Glycopep.

Selon un autre mode de réalisation, le support solide fonctionnalisé □-Z présente des groupements lieurs qui sont des groupements de type trityl-alcool, c'est à dire des groupements OH-Trt-CO-NH-, où le triphénylméthyle (Trt) est éventuellement substitué par un ou plusieurs substituants, notamment choisis parmi les substituants chlore, méthoxy, méthyle, fluor et cyano. Des supports solides présentant de tels groupements lieurs ont été décrits par exemple dans Quibell, JACS 1995, 117, 11656-11668, et sont disponibles commercialement, par exemple dans la gamme ChemMatrix® de supports solides polyéthylèneglycol.

L'utilisation de supports solides de ce type nécessite une étape préliminaire d'activation du support solide :
- soit avec un agent bromé (notamment bromure d'acétyle) pour modifier les groupements lieurs sous la forme Br-Trt-CO-NH- ;
- soit avec un agent chloré (notamment chlorure d'oxalyle) pour modifier les groupements lieurs sous la forme Cl-Trt-CO-NH- ;
- soit avec un acide comme l'acide trifluoroacetique, pour modifier les groupements lieurs sous la forme H₂O⁺-Trt-CO-NH- ;
- soit avec BF₃.Et₂O, pour modifier les groupements lieurs sous la forme BF₃ :HO-Trt-CO-NH-. selon Singh, S. et al., J. Org. Chem. 2004, 69, 4551-4554.

Une étape d'activation de ce type est décrite par exemple dans Harre et al., Reactive & functional polymers 1999, 41, 111-114.

La préparation de la résine polymère fonctionnalisée s'effectue en couplant le composé amine de formule :

(III) NH(CH₂-CH₂-Se-H)₂

au support solide fonctionnalisé, □-Z, ci-dessus. Le composé amine (III) peut lui-même être obtenu par déprotection du composé amine de formule (IIIa) ci-dessus, dans lequel G₁ est un groupement protecteur du sélénium.

Le couplage s'effectue en milieu acide afin de limiter les risques de démasquage de l'amine secondaire, ce qui induirait des réactions secondaires.

Les groupements chlorure de trityle en excès sont de préférence neutralisés, par exemple avec du méthanol. Il est alors important d'ajouter une base pour neutraliser le HCl formé.

On peut prévoir que la préparation de la résine polymère fonctionnalisée fasse partie intégrante du procédé de fabrication en phase solide des polypeptides de formule (I) et (I*'*).

Alternativement, la résine polymère fonctionnalisée peut être préparée à l'avance et de manière distincte, afin d'être prête à l'emploi dans le cadre du procédé de fabrication en phase solide des peptides de formule (I) et (I').

L'utilisation de supports solides de ce type peut permettre d'utiliser des squelettes de résine de type polyéthylèneglycol ou polyéthylèneglycol-polystyrène, plus adaptés à la préparation de longs polypeptides que les résines de type polystyrène.

De préférence, les particules de résine présentent un Dv50 compris entre 1 et 1000 µm. Le Dv50 est défini comme étant le 50^{ème} centile de la distribution de taille des particules, c'est-à-dire que 50 % des particules ont une taille inférieure au Dv50 et 50 % ont une taille supérieure au Dv50. De manière générale, le Dv50 est caractéristique du profil granulométrique (distribution volumétrique) des particules, et il peut être déterminé par granulométrie laser (pour une taille inférieure à 200 µm), ou par tamisage (pour une taille supérieure à 200 µm).

Un autre objet de l'invention concerne un peptide greffé sur une résine polymère fonctionnalisée présentant la structure (VII) ou (VII') : 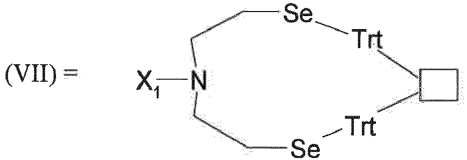 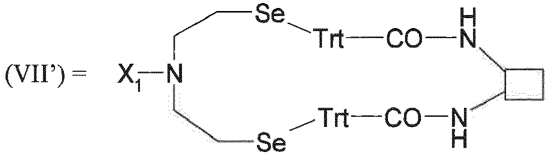 dans lesquelles,
□ représente un support solide,
Trt représente un groupement triphénylméthyle éventuellement substitué par un ou plusieurs substituants, notamment choisi parmi les substituants chlore, méthoxy, méthyle, fluor et cyano,
X₁ représente un fragment peptidique fonctionnalisé sur son extrémité C-terminale.

### Procédé d'obtention des peptides de formules (I) et (I') en phase solide

Un autre objet de l'invention concerne un procédé de fabrication en phase solide des peptides (I) et (I') précédemment décrit.

Ce procédé en phase solide comprend une phase de fonctionnalisation suivie par une phase de synthèse peptidique.

La première étape comprend la fourniture d'une résine polymère fonctionnalisée présentant la structure : 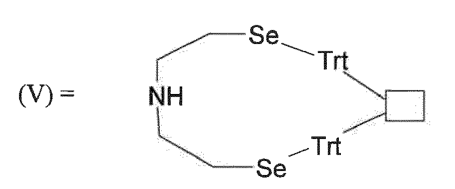 Ou 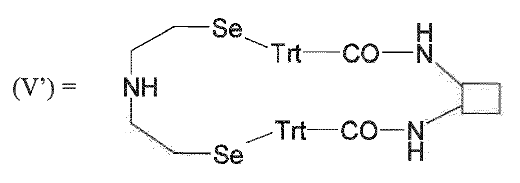 dans lesquelles,
□ représente un support solide,
Trt représente un groupement triphénylméthyle éventuellement substitué par un ou plusieurs substituants, notamment choisi parmi les substituants chlore, méthoxy, méthyle, fluor et cyano.

La seconde étape du procédé comprend le greffage d'un acide aminé G₃ - AA sur la résine polymère fonctionnalisée (V) ou (V') obtenue à la première étape pour conduire au composé de structure (VI) : 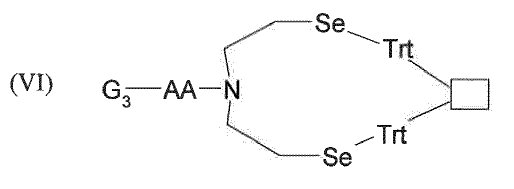 Ou de structure (VI') : 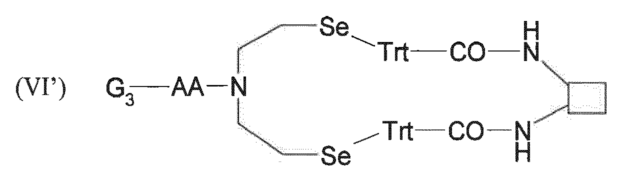 dans lesquelles,
□ et Trt ont la même signification que dans le composé (V) ou (V'),
AA représente un résidu d'acide aminé comportant éventuellement un ou plusieurs groupements protecteurs ;
G₃ représente un atome d'hydrogène ou un groupement protecteur de la fonction amine N-terminale de AA.

De préférence l'acide aminé AA est protégé à son extrémité N-terminale par un groupement protecteur G₃ labile en présence de base, de préférence choisi parmi les groupements 2-(4-nitrophénylsulfonyl)éthoxycarbonyl (NSC) ou Fmoc.

De préférence, l'acide aminé AA comporte des groupements protecteurs sur la totalité ou une partie (de préférence la totalité) des fonctions présentes sur sa chaîne latérale, et notamment les fonctions acide carboxylique (acides aspartique et glutamiques), amine (pour la lysine), alcool (pour la sérine ou la thréonine), phénol (pour la tyrosine), thiol ou sélénol (pour la cystéine ou sélénocystéine), guanidine (pour l'arginine) et imidazole (pour l'histidine). De tels groupements protecteurs sont connus de l'homme du métier. On pourra se reporter par exemple à l'ouvrage de référence Protective groups in organic synthesis, 2ème édition, T. Greene et P. Wuts, John Wiley & Sons, Inc.

De préférence, l'acide aminé est activé en présence d'HATU (2-(1H-7-Azabenzotriazol-1-yl)--1,1,3,3-tetramethyl uronium hexafluorophosphate Methanaminium), de PyBOP ou de BOP ou de manière plus particulièrement préférée en présence de PyBROP, ou encore sous forme d'un halogénure, notamment fluorure (c'est-à-dire qu'un atome de fluor est lié au groupement acyle du résidu d'acide aminé).

L'acide aminé réagit avec la fonction amine secondaire présente sur le support solide fonctionnalisé pour former une liaison amide. Après couplage de l'acide aminé, on peut éventuellement procéder à la déprotection de celui-ci.

Ainsi, l'étape de fonctionnalisation consiste dans ce mode de réalisation à créer un support solide amorce à partir d'un support solide préalablement fonctionnalisé.

La troisième étape comprend la synthèse peptidique à partir de l'acide aminé AA greffé sur la résine polymère pour conduire au composé (VII) ou (VII') :

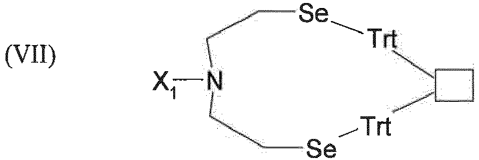

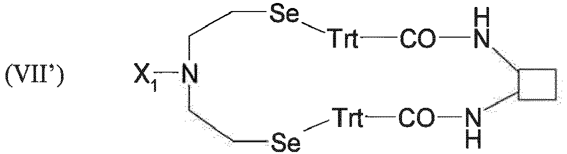

L'étape de synthèse peptidique est effectuée de manière analogue à ce qui a déjà été décrit précédemment, concernant la synthèse peptidique en phase liquide, l'amorce initiale étant fournie par l'acide aminé AA greffé sur le support solide.

Enfin, une fois la synthèse peptidique terminée, on prévoit une réaction de séparation (ou clivage) du polypeptide de son support, par coupure de la liaison entre Trt et Se pour conduire au composé (I) ou (I'). De préférence, la coupure s'effectue avec une solution d'acide trifluoroacétique comprenant les pièges à carbocations connus de l'homme du métier comme l'eau, le triisopropylsilane, le thiophénol, le thioanisole, l'anisole, le diméthylsulfure, etc...

On peut également éventuellement prévoir les déprotections adéquates des chaînes latérales des acides aminés pour l'obtention du peptide fonctionnalisé de formule (I) ou (I').

Comme pour le procédé en phase liquide, il est avantageux de prévoir à ce stade une étape de purification du composé, par exemple par chromatographie en phase liquide.

### Procédé de ligation native

Un autre objet de l'invention concerne un procédé de fabrication d'un polypeptide de formule (VIII) :

(VIII) X₁-C₁-X₂-C₂-...-Cᵢ₋₁-Xᵢ-...-Cₙ₋₁-Xₙ

dans lequel,
X₁, X₂,..., Xᵢ,..., Xₙ sont des fragments peptidiques,
C₁, C₂, ..., Cᵢ₋₁, ..., Cₙ₋₁ sont des résidus d'acides aminés portant une fonction thiol, ou sélénol
n est un entier supérieur ou égal à 2,
i est un nombre entier quelconque allant de 1 à n,
ce procédé comprenant une ou plusieurs étapes de réaction entre un composé de formule (IXᵢ) :

(IXᵢ) X'ᵢ-N(CH₂CH₂-Se-H)₂

dans laquelle :
X'₁ représente X₁
et X'ᵢ représente Cᵢ₋₁-Xᵢ pour i > 1
et un composé de formule (Xᵢ₊₁) :

(Xᵢ₊₁) Cᵢ₋Xᵢ₊₁-...-Cₙ₋₁-Xₙ

pour former un composé de formule (Xᵢ) :

(Xᵢ) X'ᵢ-Cᵢ-Xᵢ₊₁-...-Cₙ₋₁-Xₙ

Le résidu Cᵢ est un résidu d'acide aminé comportant une fonction thiol ou sélénol. Cette fonction thiol ou sélénol peut être en particulier une fonction béta-amino thiol ou béta-amino sélénol (auquel cas le résidu Cᵢ représente de préférence le résidu cystéine ou le résidu sélénocystéine), une fonction gamma-amino thiol ou gamma-amino sélénol (auquel cas le résidu Cᵢ représente de préférence le résidu homocystéine ou homosélénocystéine).

L'invention permet de produire des polypeptides en enchaînant successivement plusieurs réactions de ligation. Ceci peut s'avérer approprié pour obtenir des polypeptides de grande taille, par exemple des polypeptides comprenant plus d'environ 100 résidus d'acides aminés. En effet, dans de tels cas la fabrication de polypeptides de formules (IXᵢ) et (Xᵢ) par synthèse directe peut avoir un faible rendement, et il est donc avantageux d'utiliser deux ou plus de deux ligations successives, afin d'avoir à synthétiser directement uniquement des polypeptides comprenant par exemple moins d'environ 50 résidus d'acides aminés.

A titre d'exemple, l'utilisation de deux ligations successives permet d'obtenir un polypeptide comprenant environ 150 résidus d'acides aminés sans avoir à synthétiser directement de polypeptide comprenant plus d'environ 50 résidus d'acides aminés ; l'utilisation de trois ligations successives permet d'obtenir un polypeptide comprenant environ 200 résidus d'acides aminés sans avoir à synthétiser directement de polypeptide comprenant plus d'environ 50 résidus d'acides aminés.

Le polypeptide de formule (VIII) peut être obtenu en utilisant plusieurs méthodes de ligation. D'autres méthodes de ligation sont par exemple des méthodes faisant intervenir des thioesters ou faisant intervenir des composés soufrés particuliers tels que décrit dans le document WO 2011/051906.

Le procédé de fabrication du polypeptide de formule (VIII) selon l'invention comprend au moins une étape de réaction entre un composé de formule (IXᵢ) et un composé de formule (Xᵢ).
Ainsi, le procédé selon l'invention permet d'obtenir un polypeptide de formule (VIII); chaque Xᵢ, pour i entier entre 1 et n, étant un fragment peptidique ; et chaque Cᵢ, pour i entier entre 1 et n, étant un résidu d'acide aminé comportant une fonction thiol ou sélénol, et notamment un résidu cystéine ou sélénocystéine selon un mode de réalisation particulier, en utilisant n-1 ligations successives.

Selon un mode de réalisation de l'invention, le composé (IXᵢ) peut être obtenu à partir du composé (IX'ᵢ) : 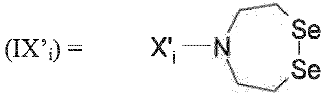 en présence d'au moins un composé réducteur des liaisons disélénium et/ou trisélénium. En effet, le polypeptide de formule (IX'ᵢ) est alors réduit in situ et fournit le polypeptide de formule (IXᵢ) pour la réaction de ligation.

Selon un mode de réalisation, le composé réducteur est choisi parmi la tris(2-carboxyéthyl)phosphine (TCEP), l'acide 4-mercaptophénylacétique (MPAA), le benzylmercaptan, le thiophénol, le DTT, le 2-mercaptoéthylsulfonate de sodium (MESNa), le sélénophénol.

De manière analogue à ce qui a été décrit précédemment pour les composés (I) et (I'), selon un mode de réalisation, les composés (IXᵢ) et (IX'ᵢ) peuvent être formés par échange, à partir d'un segment SEA de structure X'ᵢ-N(CH₂CH₂-S-G₅)₂ ou d'un thioester de structure X'ᵢ-S-R₁ à l'aide des composés séléniés de structure (III') ou (III"), éventuellement en présence de composés réducteur des liaisons disulfure et/ou disélénium, G₅ représentant H, un groupement protecteur des fonctions thiol ou une liaison disulfure et R₁ représentant H ou un groupement protecteur du soufre.

Dans le cas où i est égal à 1, les composés (IXᵢ) et (IX'ᵢ) correspondent respectivement aux composés (I) et (I').

De manière générale, la réaction de ligation à partir du polypeptide de formule (IX'ᵢ) en tant que réactif peut être plus pratique à mettre en oeuvre que la réaction de ligation directement avec le polypeptide de formule (IXᵢ). En effet, le polypeptide de formule (IXᵢ) a naturellement tendance à s'oxyder en le polypeptide de formule (IX'ᵢ), notamment sous l'action de l'oxygène de l'air. Par exemple, il est possible que le polypeptide de forme ouverte (IXᵢ) s'oxyde dans le temps lorsqu'il est stocké sous forme lyophilisée. En d'autres termes, une préparation du polypeptide de formule (IXᵢ) contient généralement nécessairement en partie le polypeptide de formule (IX'ᵢ). La présence de ces deux formes peut compliquer la caractérisation et la purification. C'est pourquoi il peut être plus simple de mettre en oeuvre la réaction de ligation par mise en contact du polypeptide de formule (IX'ᵢ) avec le polypeptide de formule (Xᵢ), le polypeptide à terminaison cyclique de formule (IX'ᵢ) étant réduit *in situ* en le polypeptide ouvert de formule (IXᵢ).

Pour les mêmes raisons, même lorsque la réaction de ligation est effectuée directement par mise en contact du polypeptide de formule (IXᵢ) avec le polypeptide de formule (Xᵢ), il est préférable d'utiliser lors de la réaction un ou plusieurs des composés réducteurs des liaisons disélénium mentionnés ci-dessus.

La réaction de ligation intervient de préférence en phase liquide, et notamment en milieu aqueux, par exemple dans un tampon phosphate. De préférence, cette réaction est effectuée à un pH compris entre 4 et 8,5, de manière plus particulièrement préférée à un pH compris entre 5 et 7,5 et idéalement à un pH voisin de 5,5.

La réaction de ligation est effectuée de préférence à une température comprise entre 0 et 50°C, et idéalement à une température d'environ 37°C. La durée de la réaction est ajustée en fonction du choix des réactifs et des autres conditions de la réaction. La durée appropriée peut également être ajustée selon les résultats d'une analyse de chromatographie liquide - spectrométrie de masse au cours de la réaction. La durée appropriée sera typiquement de quelques heures à quelques jours.

Chacun des polypeptides de formule (IXᵢ) et (Xᵢ) est de préférence présent à une concentration comprise entre 0,01 et 50 mM, lors de la réaction. Le rapport de concentration molaire entre les polypeptides de formule (IXᵢ) et (Xᵢ) lors de la réaction est de préférence compris entre 2:3 et 3:2.

La réaction de ligation décrite ci-dessus peut être suivie d'une étape de purification du polypeptide de formule (Xᵢ) obtenu, par exemple par chromatographie en phase liquide ou par toute autre technique usuelle.

La cinétique de ligation selon la présente invention est significativement supérieure à la cinétique de ligation obtenue avec des composés soufrés décrits dans le document WO 2011/051906 ou avec des thioesters comme ceux enseignés par Kent et Dawson.

Du fait de la rapidité de la ligation SeEA, elle peut être réalisée en présence d'autres groupements capables de participer à une réaction de ligation native, comme les segments SEA ou thioester.

La combinaison des ligations SeEA et SEA constitue un outil très performant pour la synthèse totale de protéines. Pour la production d'une protéine qui doit être synthétisée par ligation de plusieurs fragments on peut par exemple mettre en oeuvre la ligation SEA pour les jonctions faciles, et la ligation SeEA pour les jonctions difficiles. En effet, la ligation SeEA permet de former facilement des jonctions encombrées, en 4 heures par exemple pour la jonction Val-Cys, alors que ce type de jonction demande des temps de ligation de 96 heures avec la ligation SEA ou et de 8 heures pour la ligation NCL.

Une autre façon intéressante d'exploiter les ligations SeEA et SEA est la ligation one-pot séquentielle SeEA-SEA. Celle-ci peut être mise en oeuvre pour lier trois fragments sans isolement et purification intermédiaire (réaction dite en un pot) lorsque les jonctions ont un encombrement stérique analogue. Un exemple de mise en oeuvre de cette variante est explicité ci-dessous.

Selon un mode de réalisation de l'invention, le procédé de ligation de l'invention permet la fabrication à partir de trois fragments peptidiques d'un polypeptide représenté par la formule (XII) :

(XII) = X₁-C₁-X₂-C₂-X₃

ledit polypeptide étant obtenu par un procédé comprenant les étapes suivantes :
a) réaction entre un composé de formule (XIII) ou (XIII') :

   (XIII) = R₂-X₁-N(CH₂-CH₂-Se-H)₂

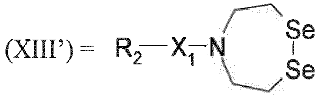

   et un composé de formule (XIV) ou (XIV') :

   (XIV) = H-C₁-X₂-N(CH₂-CH₂-S-H)₂

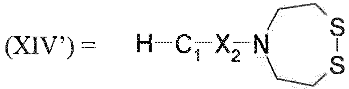

   pour former le composé de formule (XV) ou (XV') :

   (XV) = R₂-X₁-C₁-X₂-N(CH₂-CH₂-S-H)₂ 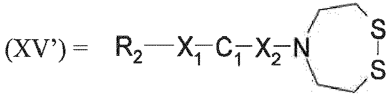
b) réaction entre un composé de formule (XV) ou (XV') et un polypeptide de formule (XVI) :

   (XVI) H-C₂-X₃

   pour former le composé de formule (XII),
   dans lesquelles,
   R₂ représente un groupement protecteur de la fonction N-terminale de X₁,
   X₁, X₂, X₃ représentent, indépendamment les uns des autres, des fragments peptidiques,
   C₁ et C₂ représentent, indépendamment l'un de l'autre, des résidus d'acides aminés porteurs d'une fonction thiol ou sélénol.

De façon analogue à ce qui est décrit précédemment pour l'obtention des composés (I) et (I'), les composés ci-dessus (XIII) et (XIII') peuvent être formés in situ, par échange à partir de réactifs de type (IIIa), (III') ou (III") en présence de composés SEA de structure R₂-X₁- SEA ou thioester de structure R₂-X₁-S-R₁, dans lequel R₁ représente H ou un alkyle ou un aryle éventuellement substitué, permettant ainsi d'accélérer la deuxième étape de ligation.

Cette réaction de ligation one-pot de trois segments peut être mise en oeuvre pour lier trois fragments sans isolement et purification intermédiaire (réaction dite « one-pot ») lorsque les jonctions ont un encombrement stérique analogue. L'encombrement des jonctions étant analogue, la ligation entre le composé (XIII) ou (XIII') et le composé (XIV) ou (XIV') sera plus rapide que la cyclisation du composé (XIV) ou (XIV') par ligation SEA intramoléculaire, ou que la polymérisation du segment (XIV) ou (XIV') par ligation SEA intermoléculaire. Les ligations SEA intramoléculaire ou intermoléculaire sont décrites dans le document WO 2011/051906.

### Production d'un polypeptide cyclique par auto-ligation native

Les principes utilisés pour la ligation native décrits ci-dessus peuvent également être utilisés pour produire des polypeptides cycliques, par auto-ligation native d'un polypeptide (ligation d'une extrémité du polypeptide avec l'autre extrémité du même polypeptide).

Un autre objet de l'invention concerne un procédé de fabrication d'un polypeptide cyclique de formule (XVII) : 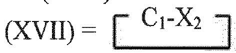 X₂ représentant un fragment peptidique, et C₁ représentant un résidu d'acide aminé comportant une fonction thiol ou sélénol, ledit procédé comprenant au moins une étape de réaction de ligation d'un polypeptide de formule (XVIII) :

(XVIII) H-C₁-X₂-N(CH₂-CH₂-Se-H)₂

avec lui-même.

Le polypeptide de formule (XVIII) comprend de préférence entre 2 et 300 résidus d'acides aminés, de préférence entre 5 et 100 résidus d'acides aminés, de manière plus particulièrement préférée entre 8 et 50 résidus d'acides aminés.

Le polypeptide de formule (XVIII) comprend un atome d'hydrogène et un résidu C₁ à l'extrémité N-terminale, C₁ ayant la signification indiquée ci-dessus. X₂ représente ici un fragment peptidique de la forme AA₁AA₂...AAₙ où n est un nombre entier supérieur ou égal à 1 et chaque AAᵢ représente un résidu d'acide aminé.

Le polypeptide de formule (XVIII) comprend de préférence uniquement des résidus d'acides aminés choisis parmi les vingt résidus d'acides aminés protéinogéniques et la sélénocystéine. Toutefois, selon un mode de réalisation particulier, le polypeptide de formule (XVIII) comprend un ou plusieurs résidus d'acides aminés non- protéinogéniques, autres que la sélénocystéine.

Les résidus d'acides aminés du polypeptide de formule (XVIII) peuvent éventuellement être protégés par des groupements protecteurs des chaînes latérales.

Il est possible d'effectuer la réaction de ligation ci-dessus en mettant en présence le polypeptide de formule (XVIII') : 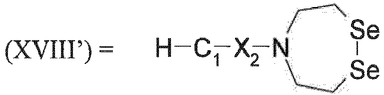 avec au moins un composé réducteur des liaisons disélénium, qui est de préférence tel que décrit ci-dessus. En effet, le polypeptide de formule (XVIII') est alors réduit *in situ* et fournit le polypeptide de formule (XVIII) pour la réaction de ligation.

De manière générale, même lorsque la réaction de ligation est effectuée directement à partir du polypeptide de formule (XVIII), il est préférable d'utiliser lors de la réaction un ou plusieurs des composés réducteurs des liaisons disélénium mentionnés ci-dessus.

En général, la cyclisation du polypeptide de formule (XVIII) n'est pas gênée par des multimérisations concurrentes, si la réaction est effectuée en conditions suffisamment diluées. On peut par exemple utiliser une concentration de polypeptide de formule (XVIII) comprise entre 0,01 et 50 mM, typiquement d'environ 1 mM (ou éventuellement entre 0,01 et 0,1 mM en cas de risques sérieux de multimérisations).

Par ailleurs, les conditions préférées de mise en oeuvre de la réaction de ligation sont les mêmes que celles décrites ci-dessus pour la réaction à partir des polypeptides de formule (IX) et (X).

La réaction de ligation décrite ci-dessus peut être suivie d'une étape de purification du polypeptide cyclique de formule (XVII) obtenu, par exemple par chromatographie en phase liquide ou par toute autre technique usuelle.

### Kit de synthèse

Un autre objet de l'invention concerne un kit de synthèse de polypeptide comprenant un ou plusieurs peptides de formules (IXᵢ) ou (IX'ᵢ), ou au moins un composé de formule (III') ou (III"), et éventuellement un ou plusieurs réactifs de couplage, de protection, de déprotection, ou des solvants.

### EXEMPLES

### Exemple 1 : Synthèse des composés 1 (Se₂EA) et 2 (Se₃EA)

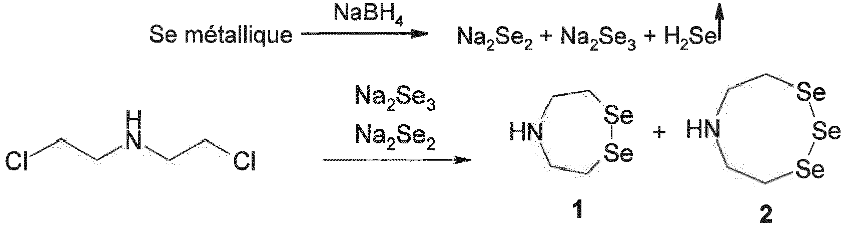

Sous balayage d'azote, de l'éthanol absolu V = 19 mL est additionné goutte à goutte, sous agitation à un mélange de sélénium Se (940 mg, 11,9 mmol, 1,5 eq) et de borohydrure de sodium (NaBH₄) (300 mg, 7,9 mmol) refroidi dans un bain de glace. Après une réaction vive, le milieu réactionnel est porté à reflux pendant 1h30. Le disélénure de sodium Na₂Se₂ formé en solution est de couleur brun-rouge.

A température ambiante, le bis(2-chloroéthyl)amine hydrochloride (847 mg, 4,7 mmol, 0,5eq) en solution dans un mélange NaOHaq (0,5 M)/EtOH (1/1) V = 3 mL est additionnée goutte à goutte pendant 10 minutes sur la solution de Na₂Se₂ et Na₂Se₃ précédente. Le milieu réactionnel est agité 45 minutes à température ambiante.

Le milieu réactionnel est alors dilué V = 10 mL par une solution de NaOHaq (0,5M) puis extrait par du CH₂C1₂ (3 x 30 mL). Les phases organiques sont alors rassemblées, séchées par du Na₂SO₄, puis concentrées jusqu' à 1/3 du volume total.

Le concentrât organique obtenu est alors extrait par une solution aqueuse d'acide trifluoroacétique (TFA) (10%) puis purifié directement par RP-HPLC : Colonne C18 X-bridge (d = 1,9 cm, L = 20 cm, 130 Ǻ, 5 µm), détection UV (λ = 215 nm), tampon A : H₂O/TFA (1:0,05% en v/v), tampon B : CH₃CN/H₂O/TFA (4:1:0,05% en v/v/v), isocratique : tampon A (3 min), puis gradient : tampon B (0 à 50% en 9 min, 25 mL/min).

Après lyophilisation, les composés **1** (Se₂EA) 230 mg (Rendement R=21 %) et les composés 2 (Se₃EA) 240 mg (Rendement R= 16 %) sont obtenus sous forme d'une poudre jaune.

### Analyse LC-MS Chromatogramme RP-HPLC des composés 1 (Se₂EA) et 2 (Se₃EA) respectivement

LC-MS : Tampon A : H₂O/TFA (1/0,1% v/v), Tampon B : CH₃CN/H₂O/TFA (4/1:0,1% v/v/v).

RP-HPLC sur une colonne C18 Xbridge BEH (300 Ǻ, 3,5 µm, 4,6 x 150 mm) en utilisant un gradient linéaire 0-100% B en 30 min, débit 1 mL/min, détection UV (λ = 215 nm).

MS : Mode Ionisation Electrospray positif, voltage cône 30V, analyseur quadripolaire.

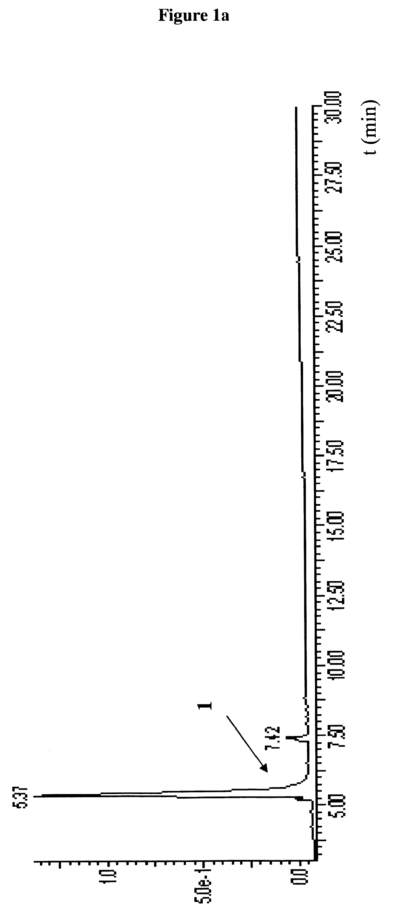

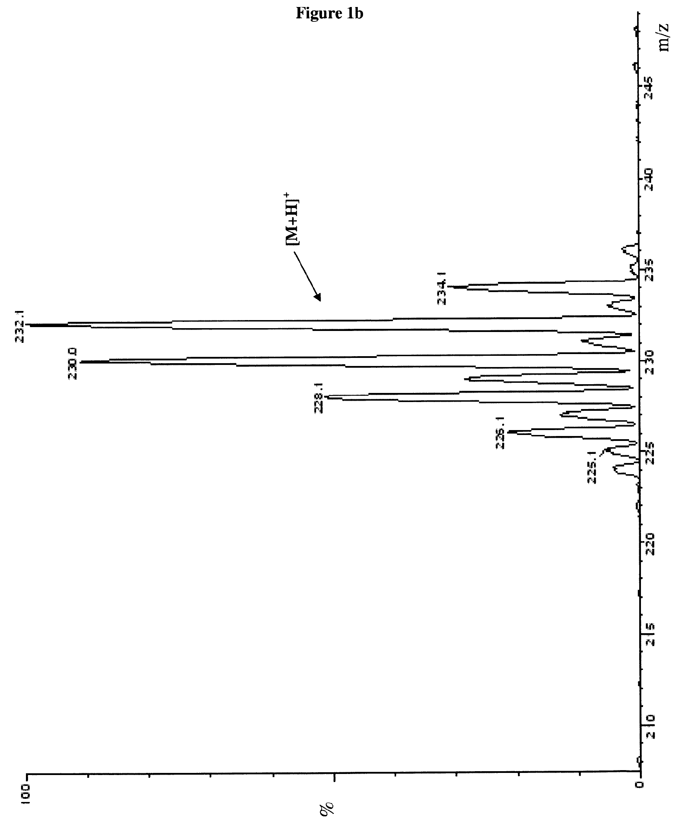

Le chromatogramme et le spectre de masse du composé 1 sont représentés sur les figures 1a et 1b respectivement.

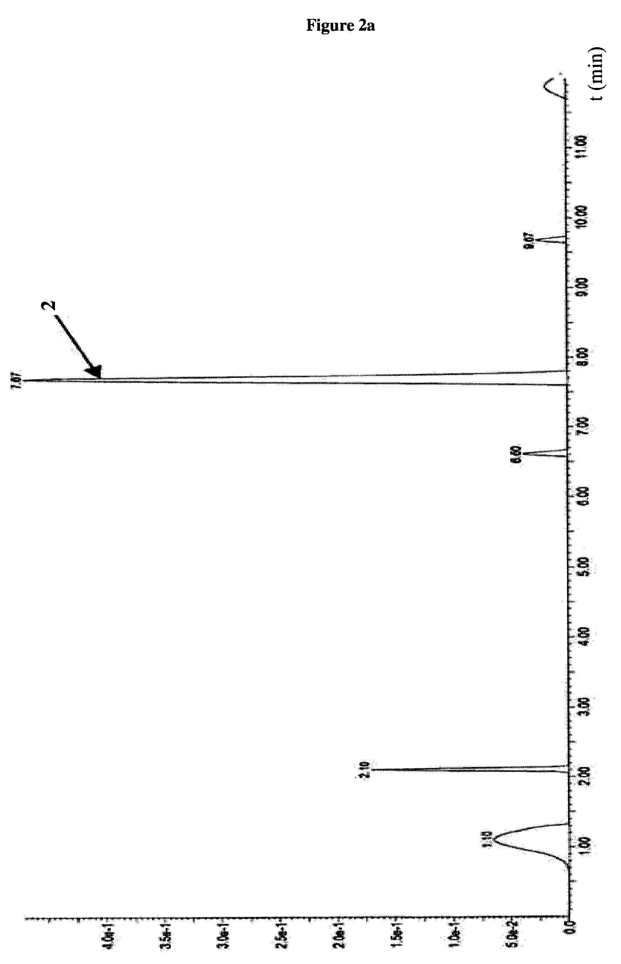

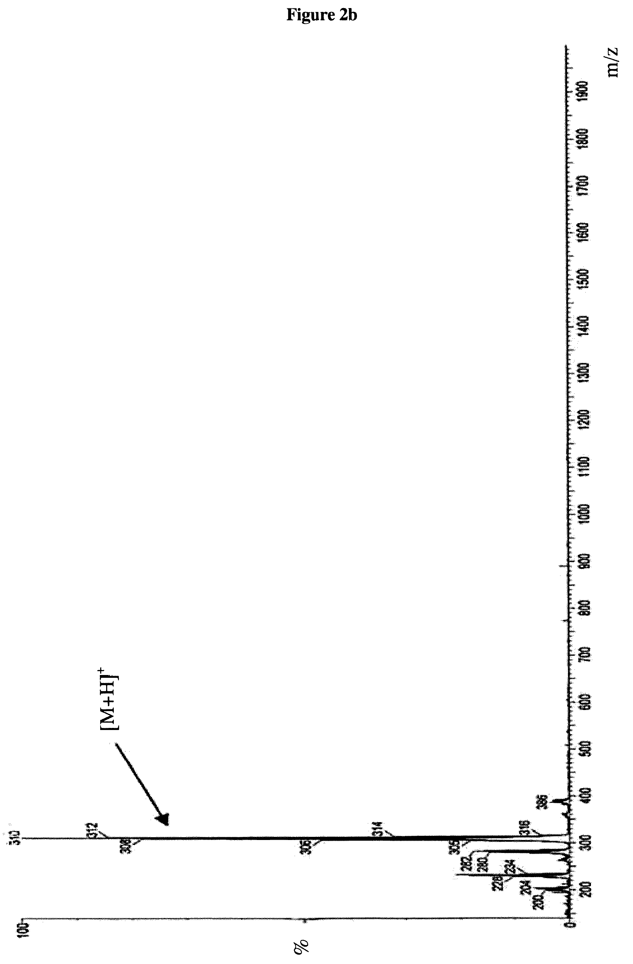

Le chromatogramme et le spectre de masse du composé 2 sont représentés sur les figures 2a et 2b respectivement.

### Exemple 2 : Synthèse et isolement des segments SeEA selon l'invention par échange, à partir de segments SEA.

### Synthèse du peptide 8 : H-ILKEPVHGA-SEA

La méthode de synthèse de ce peptide est décrite dans le document Ollivier, N.; Dheur, J.; Mhidia, R.; Blanpain, A.; Melnyk, O. Organic letters 2010, 12, 5238-41.

### Synthèse du peptide 9 : H-ILKEPVHGA-SeEA

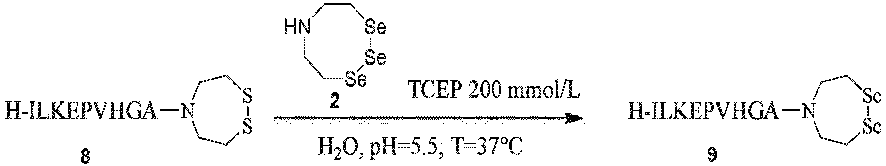

La réaction est réalisée dans une boite à gant (la concentration en dioxygène est inférieure à 50 ppm) sous atmosphère d'azote. Une solution de TCEP à 200 mmol/L est préparée dans un tampon phosphate 0,1M à pH = 7,2. Le pH de la solution TCEP/MPAA obtenue est ajusté à pH = 5,5.

Le peptide **8** (3,8 mg, 3 µmol) et du linker Se₃EA 2 (8,4 mg, 28 µmol, 10 eq) sont dissous dans la solution précédente de TCEP V = 297 µL. Le milieu réactionnel est agité 24 h à 37°C.

En fin de réaction, le milieu réactionnel est dilué V = 2 mL par une solution de TFA (0,1%) puis purifié directement par chromatographie liquide haute performance en phase inverse (RP-HPLC) (Colonne C18 Nucleosil (d=1 cm, L=20 cm, 120 Ǻ, 5 µm), détection UV (λ = 215 nm), tampon A : H₂O/TFA (1:0,05% en v/v), tampon B : CH₃CN/H₂O/TFA (4:1:0,05% en v/v/v), gradient : tampon B (0 à 20% en 5 min puis 20 à 42% en 60 min, 6 mL/min)). 1,96 mg de peptide 9 sont obtenus (rendement 62%).

### Analyse LC-MS Chromatogramme RP-HPLC du peptide 9

LC-MS : Tampon A : H₂O/TFA (1/0,1% v/v), Tampon B : CH₃CN/H₂O/TFA (4/1:0,1%v/v/v).

RP-HPLC sur une colonne C18 Xbridge BEH (300 Ǻ, 3,5 µm, 4,6 x 150 mm) en utilisant un gradient linéaire 0-100% B en 30 min, débit 1 mL/min, détection UV (λ = 215 nm).

MS : Mode Ionisation Electrospray positif, voltage cône 30V, analyseur quadripolaire.

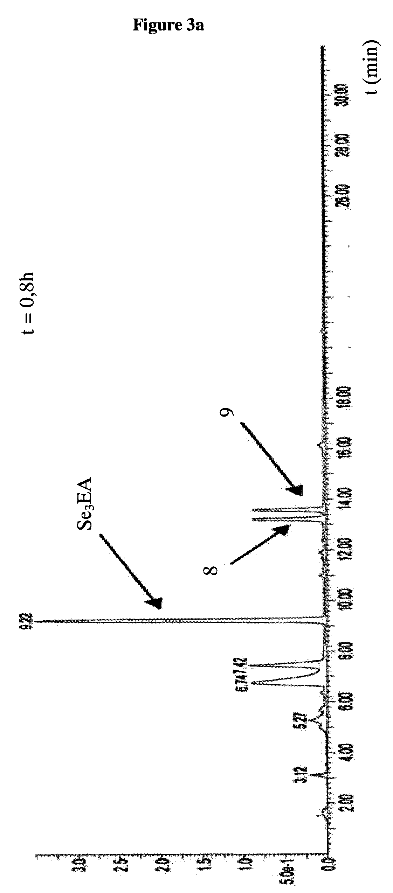

La figure 3a correspond au chromatogramme de la solution après un temps de réaction t de 0,8 h. Pour t = 0,8 h, la réaction d'échange n'est pas terminée, en effet, le pic correspondant au peptide **8** est encore présent en quantité significative.

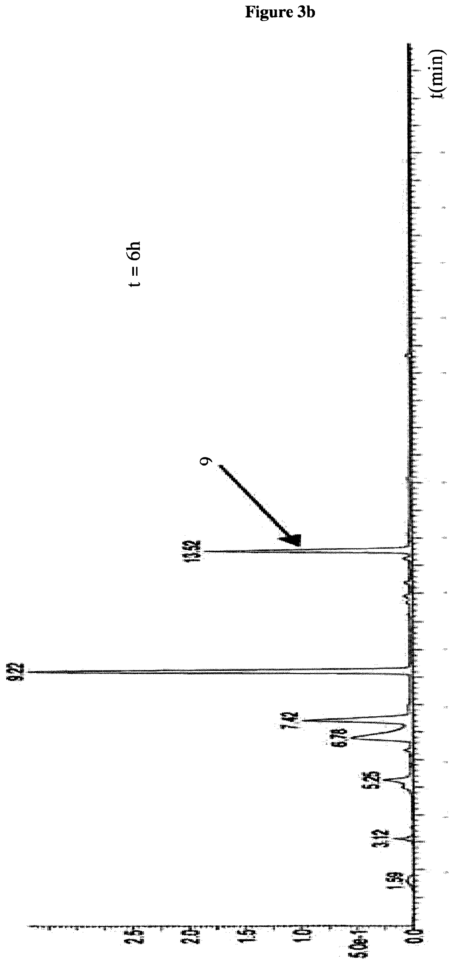

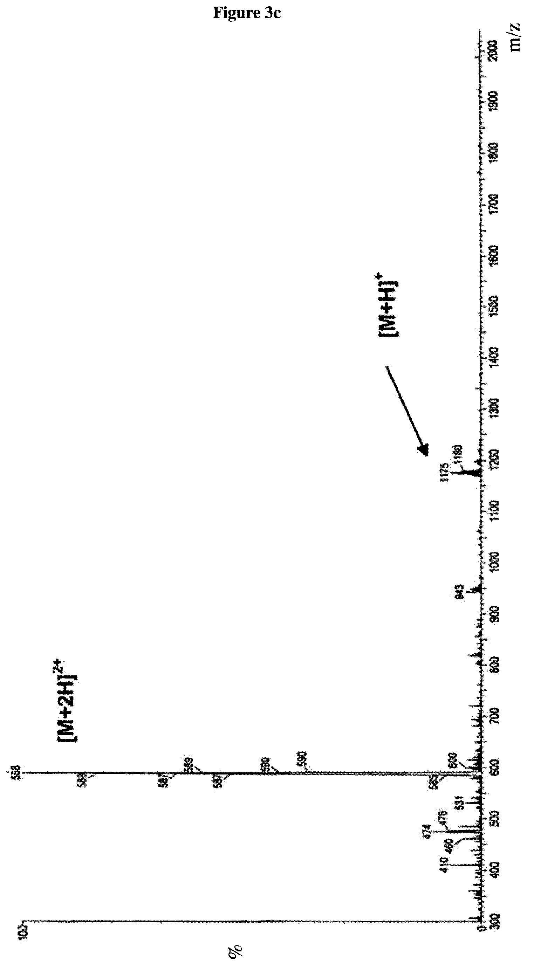

La figure 3b correspond au chromatogramme de la solution après un temps de réaction t de 6 h et la figure 3c est le spectre de masse correspondant. Ce chromatogramme montre que la réaction d'échange est terminée.

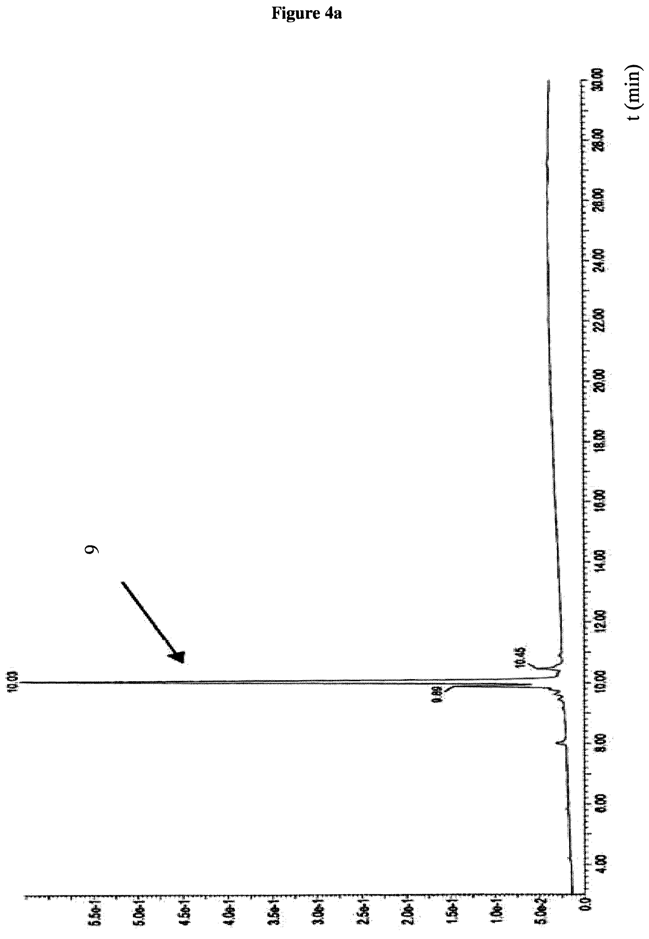

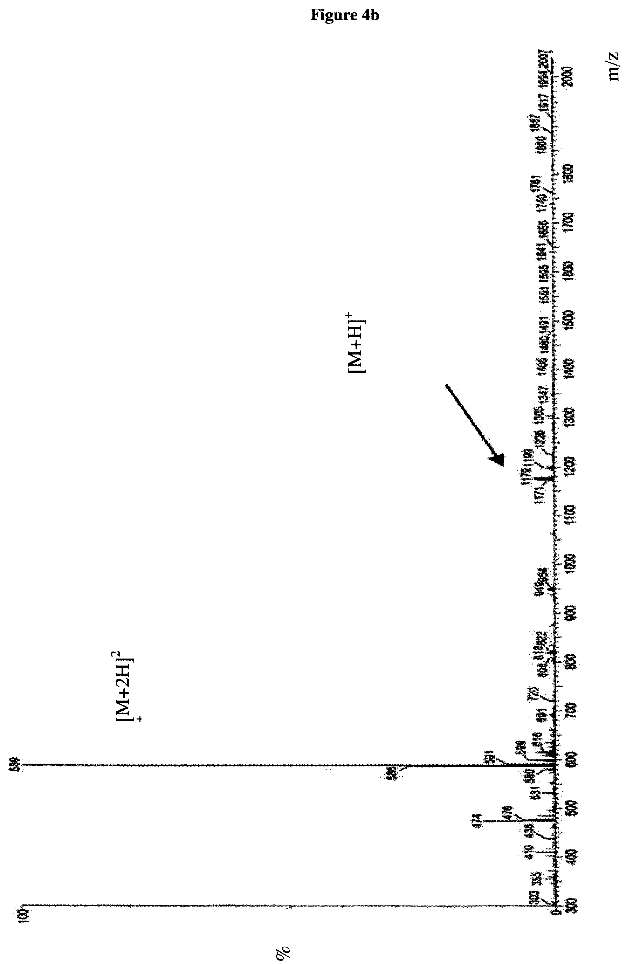

Les figures 4a et 4b correspondent au chromatogramme et au spectre de masse du peptide **9** après purification.

### Exemple 2bis: Synthèse et isolement des segments SeEA de grande taille selon l'invention par échange, à partir de segments SEA.

### Synthèse du peptide 15 : H-IRNCIIGKGRSYKGTVSITKSGIK-SEA

La méthode de synthèse de ce peptide est décrite dans le document Ollivier, N.; Dheur, J.; Mhidia, R.; Blanpain, A.; Melnyk, O. Organic letters 2010, 12, 5238-41.

### Synthèse du peptide 16 : H-IRNCIIGKGRSYKGTVSITKSGIK-SeEA

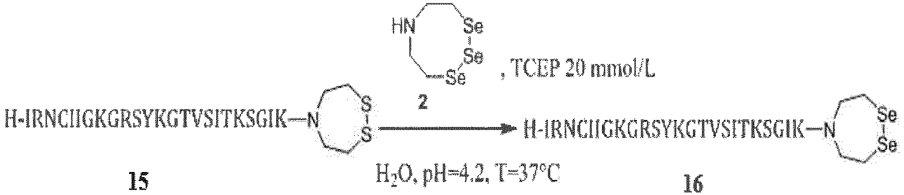

La réaction est réalisée sous atmosphère d'azote. Une solution de TCEP à 20 mmol/L est préparée dans un tampon acétate 0,1M à pH=5,5. Le pH de la solution TCEP obtenue est ajusté à pH= 4,2.

Le peptide **15** (4 mg, 1,4 µmol) et du linker Se₃EA **2** (4,4 mg, 14,3 µmol, 10 eq) sont dissous dans la solution précédente de TCEP V= 719 µl. Le milieu réactionnel est agité 24 h à 37°C.

En fin de réaction, le milieu réactionnel est dilué V=3 mL par une solution de TFA (0,1%) puis purifié directement par chromatographie liquide haute performance en phase inverse (RP-HPLC) (Colonne C18 Nucleosil (d=1 cm, L=20 cm, 120 Ǻ, 5 µm), détection UV (λ = 215 nm), tampon A : H₂O/TFA (1:0,05% en v/v), tampon B : CH₃CN/H₂O/TFA (4:1:0,05% en v/v/v), gradient : tampon B (0 à 20% en 5 min puis 20 à 42% en 60 min, 6 mL/min)). 1,8 mg de peptide 16 sont obtenus (Rendement 38%).

### Analyse LC-MS Chromatogramme RP-HPLC du peptide 16

LC-MS : Tampon A : H₂O/TFA (1/0,1% v/v), Tampon B : CH₃CN/H₂O/TFA (4/1:0,1% v/v/v).

RP-HPLC sur une colonne C18 Xbridge BEH (300 Ǻ, 3,5 µm, 4,6 x 150 mm) en utilisant un gradient linéaire 0-100% B en 30 min, débit 1 mL/min, détection UV (λ = 215 nm).

MS : Mode Ionisation Electrospray positif, voltage cône 30V, analyseur quadripolaire.

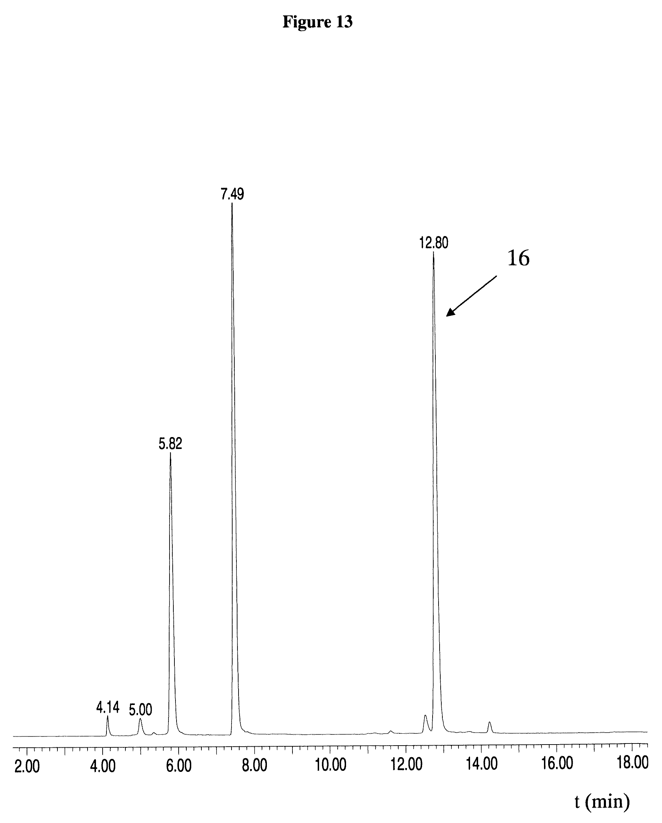

La figure 13 correspond au chromatogramme de la solution après un temps de réaction t de 24 h. Le pic correspondant au peptide **16** est bien présent et indique que la réaction d'échange est terminée.

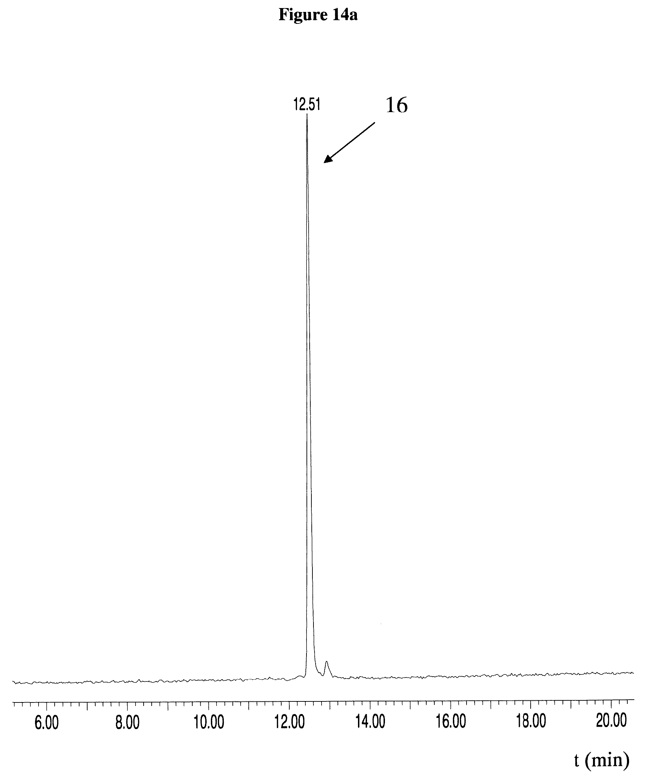

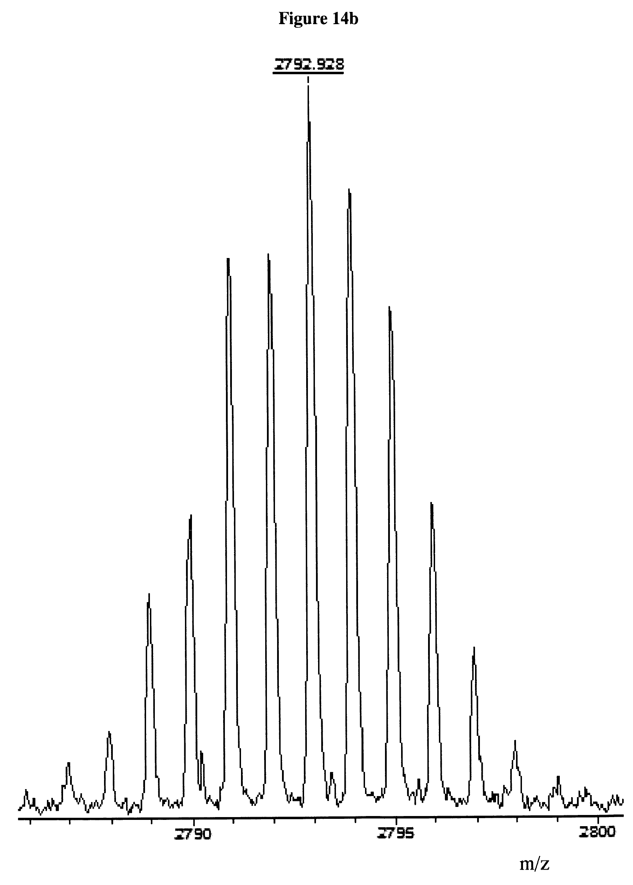

Les figures 14a et 14b correspondent respectivement au chromatogramme et au spectre de masse du peptide **16** après purification.

### Exemple 3 : Ligation native avec des composés à base de sélénium selon l'invention ou à base de soufre selon l'art antérieur

### Synthèse du peptide 3, SEQ ID NO : 1 Ac-GFGQGFGG-OH

La méthode de synthèse de ce peptide est décrite dans le document Ollivier, N.; Dheur, J.; Mhidia, R.; Blanpain, A.; Melnyk, O. Organic letters 2010, 12, 5238-41.

### Synthèse du peptide 4 : Ac-GFGQGFGG-SeEA

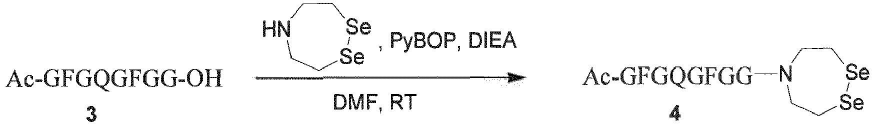

Le peptide **3** (50 mg, 0,07 mmol) et la molécule **1** (30 mg, 0,13 mmol, 2 eq) sont dissous dans V = 3,5 mL de DMF (*N,N*-diméthylformamide) suivi par l'ajout de benzotriazol-1-yloxy-tripyrrolidinophosphoniumhexafluorophosphate (PyBOP) (68 mg, 0,14 mmol, 2 eq). De la *N,N-*diisopropylethylamine (DIEA) est alors additionnée (45 µL, 0,28 mmol , 4 eq). Le milieu réactionnel est agité 1,5 heures à température ambiante puis précipité dans un mélange froid d'éther diéthylique/heptane (75 mL, 1:1 v/v), centrifugé, puis dissous dans un minimum d'eau et lyophilisé.

Le peptide **4** brut est purifié directement par chromatographie liquide haute performance en phase inverse (RP-HPLC) (Colonne C18 Nucleosil (d = 1 cm, L = 20 cm, 120 Ǻ, 5 µm), détection UV (λ = 215nm), tampon A : H₂O/TFA (1:0,05% en v/v), tampon B : CH₃CN/H₂O/TFA (4:1:0,05% en v/v/v), gradient : tampon B (0 à 20% en 5 min puis 20 à 42% en 60 min, 6 mL/min)). 15 mg de peptide **4** sont obtenus (Rendement 24 %).

### Analyse LC-MS Chromatogramme RP-HPLC du peptide 4

LC-MS : Tampon A : H₂O/TFA (1/0,1% v/v), Tampon B : CH₃CN/H₂O/TFA (4/1:0,1% v/v/v).

RP-HPLC sur une colonne C18 Xbridge BEH (300 Å, 3,5 µm, 4,6 x 150 mm) en utilisant un gradient linéaire 0-100% B en 30 min, débit 1 mL/min, détection UV (λ = 215 nm).

MS : Mode Ionisation Electrospray positif, voltage cône 30V, analyseur quadripolaire.

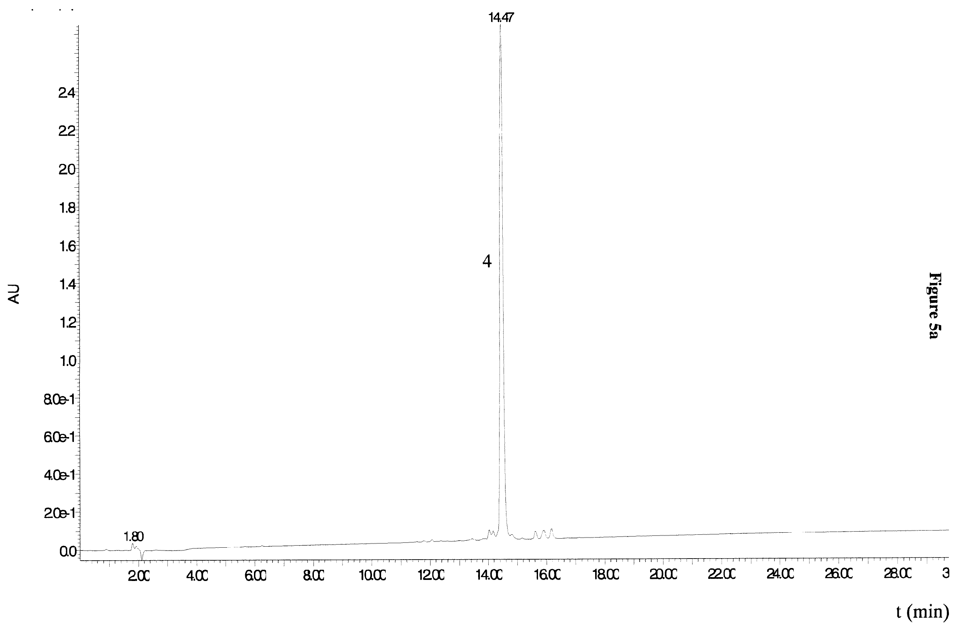

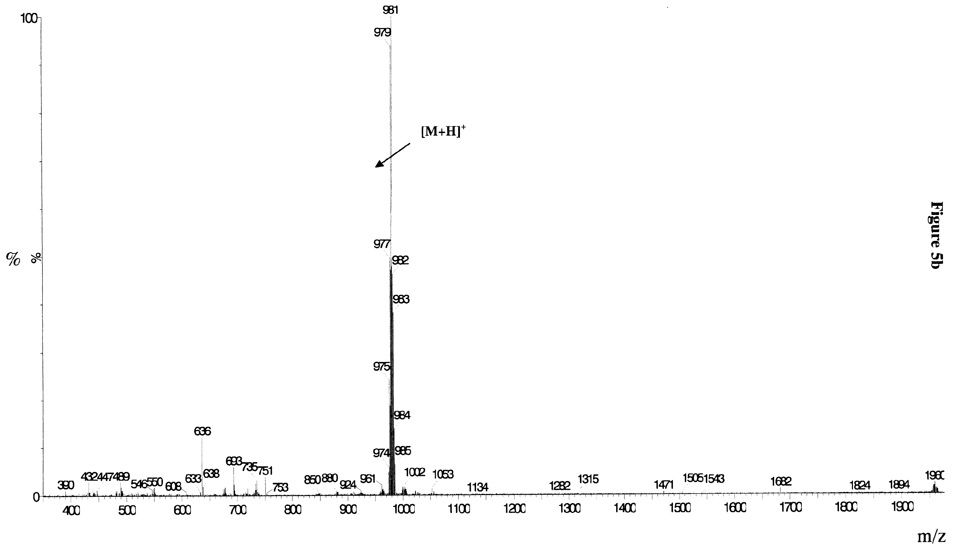

Les figures 5a et 5b représentent respectivement le chromatogramme et le spectre de masse du peptide **4** après purification.

### Synthèse du peptide 5 : Ac-GFGQGFGG-SEA

La méthode de synthèse de ce peptide est décrite dans le document Ollivier, N.; Dheur, J.; Mhidia, R.; Blanpain, A.; Melnyk, O. Organic letters 2010, 12, 5238-41.

### Synthèse du peptide 6, SEQ ID NO : 2 H-CILKEP VHGA -NH₂

La méthode de synthèse de ce peptide est décrite dans le document Ollivier, N.; Dheur, J.; Mhidia, R.; Blanpain, A.; Melnyk, O. Organic letters 2010, 12, 5238-41.

### Synthèse du peptide 7, SEQ ID NO : 3 Ac-GFGQGFGGCILKEPVHGA-NH₂ par ligation SeEA. Comparaison de la réactivité du peptide 4 Ac-GFGQGFGG-SeEA et du peptide 5 Ac-GFGQGFGG-SEA

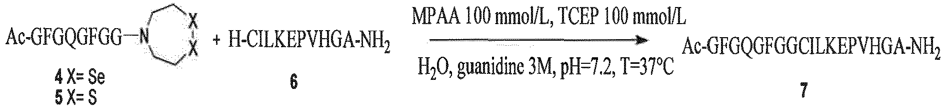

### a- Cinétique et rendement de ligation du peptide 4 Ac-GFGQGFGG-SeEA avec le peptide 6 H-CILKEPVHGA-NH₂

La ligation SeEA du peptide **4** avec le peptide **6** est réalisée dans une boite à gant (concentration en dioxygène inférieure à 50 ppm) sous atmosphère d'azote. Une solution de guanidine 3 M est préparée dans un tampon phosphate 0,2M à pH = 7,3. De la tris(2-carboxyéthyl)phosphine (TCEP, 28,6 mg, 0,1 mmol) et de l'acide 3-mercaptophénylacétique (MPAA, 16,8 mg, 0,1 mmol) sont alors additionnés. Le pH de la solution TCEP/MPAA/Guanidine obtenue est ajusté à pH = 7,2.

Les peptides **4** (4,9 mg, 5 µmol) et **6** (10,6 mg, 7,6 µmol, 1,5 eq) sont dissous dans la solution précédente de TCEP/MPAA/Guanidine (1,5 mL). Le milieu réactionnel est agité 24 h à 37°C.

La réaction de ligation terminée, le milieu réactionnel est dilué par de l'eau V = 3 mL puis acidifié par une solution de TFA (10%) V = 1 mL puis le MPAA est extrait à l'ether par 3 fois 6,5 mL.

Le produit de ligation **7** en phase aqueuse est purifié directement par chromatographie liquide haute performance en phase inverse (RP-HPLC) (Colonne C18 Nucleosil (d = 1 cm, L = 20 cm, 120 Ǻ, 5 µm), détection UV (λ = 215□ nm), tampon A : H₂O/TFA (1:0,05% en v/v), tampon B : CH₃CN/H₂O/TFA (4:1:0,05% en v/v/v), gradient : tampon B (0 à 20% en 5 min puis 20 à 42% en 60 min , 6 mL/min). 5,6 mg de peptide **7** sont obtenus (Rendement 54 %).

### Analyse LC-MS Chromatogramme RP-HPLC du peptide 7

LC-MS : Tampon A : H₂O/TFA (1/0,1% v/v), Tampon B : CH₃CN/H₂O/TFA (4/1:0,1% v/v/v).

RP-HPLC sur une colonne C18 Xbridge BEH (300 Ǻ, 3,5 µm, 4,6 x 150 mm) en utilisant un gradient linéaire 0-100% B en 30 min, débit 1 mL/min, détection UV (λ = 215 nm).

MS : Mode Ionisation Electrospray positif, voltage cône 30V, analyseur quadripolaire.

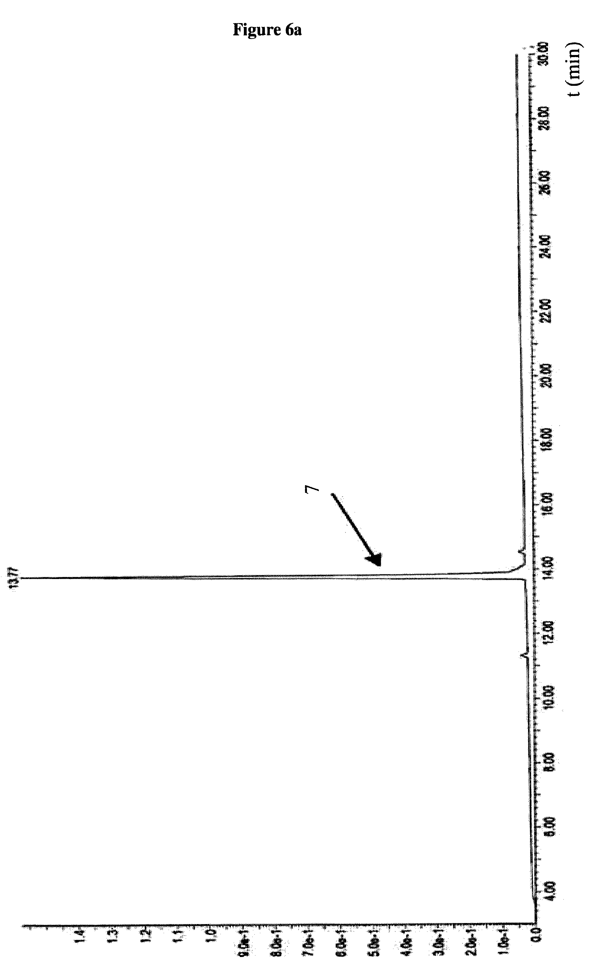

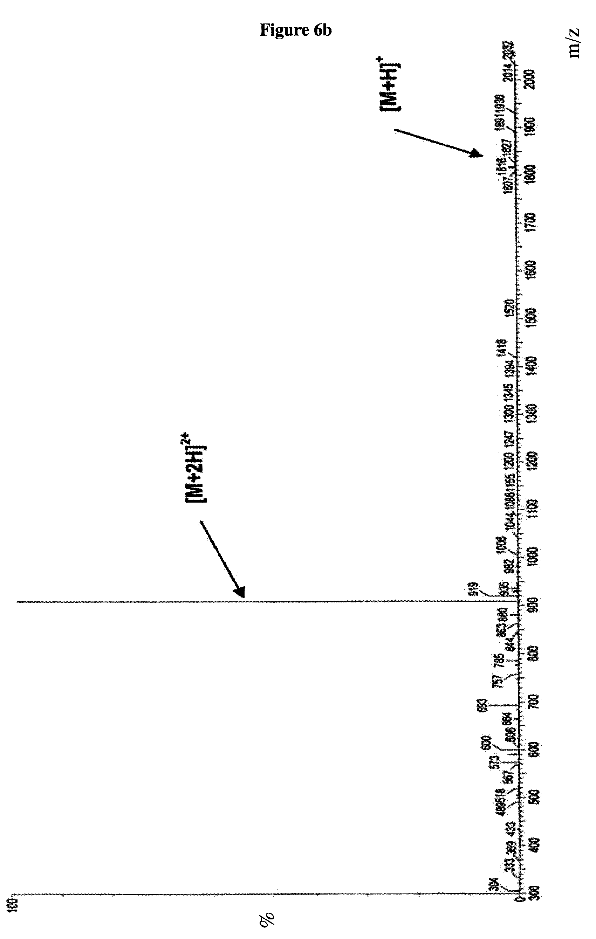

Les figures 6a et 6b représentent respectivement le chromatogramme et le spectre de masse du peptide 7 après purification.

### b- Cinétique de ligation du peptide 5 Ac-GFGQGFGG-SEA avec le peptide 6 H-CILKEPVHGA-NH₂

La réaction de ligation SEA du peptide **5** avec le peptide **6** est réalisée strictement dans les mêmes conditions.

### Comparaison des cinétiques

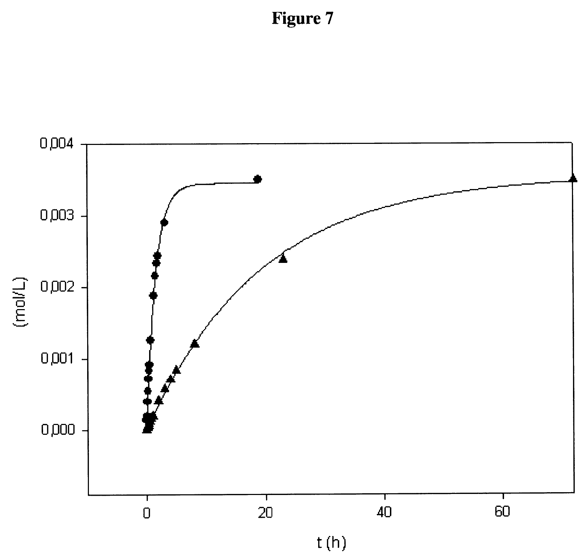

La figure 7 représente une comparaison des cinétiques de réaction de la ligation pour la formation du peptide **7,** avec un composé à base de sélénium (peptide **4)** selon l'invention, courbe représentée par le symbole • ou avec un composé à base de soufre (peptide **5)** selon l'état de la technique, courbe représentée par le symbole A.

La figure 7 montre une vitesse de réaction plus grande pour la réaction de ligation selon l'invention, courbe représentée par le symbole •.

### Exemple 4 : Comparaison des cinétiques de ligations pour les méthodes SeEA. SEA et NCL (chimie des thioesters)

### Synthèse du peptide 15a : H-ILKEPVHGV-SEA

La méthode de synthèse de ce peptide est décrite dans le document Ollivier, N.; Dheur, J.; Mhidia, R.; Blanpain, A.; Melnyk, O. Organic letters 2010, 12,5238-41*.*

### Synthèse du peptide 15b : H-ILKEPVHGV-SeEA

A partir du peptide **15a,** la méthode de synthèse du peptide **15b** est strictement identique à celle utilisée pour la synthèse du peptide **9.**

### Synthese du peptide 15c : H-ILKEPVHGV-MPA (MPA = acide 3-mercapto propionique)

La méthode de synthèse de ce peptide est décrite dans le document Dheur, J.; Ollivier, N.; Vallin, A.; Melnyk, O. Journal of Organic Chemistry 2010, 76,3194-3202.

### Comparaison des cinétiques de ligation du peptide 15a/15b/15c H-ILKEPVHGV-R avec le peptide 6 H-CILKEPVHGA-NH₂ pour les méthodes de ligations SeEA, SEA et NCL

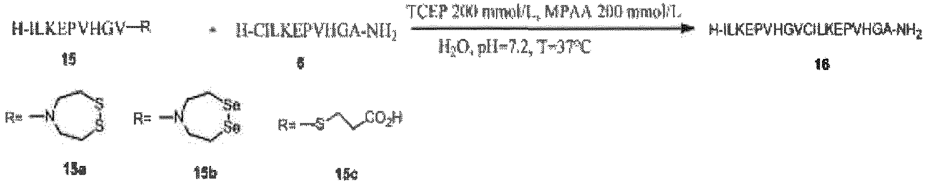

Les différentes ligations du peptide 15a ou 15b ou **15c** avec le peptide 6 sont réalisées séparément dans une boite à gant ([O₂] <50 ppm) sous atmosphère d'azote. Une solution de TCEP à 200 mmol/L est préparée dans un tampon phosphate 0,1M à pH=7,2. L'acide 3-mercaptophénylacétique (MPAA 33 mg, 0,2 mmol) est alors additionné. Le pH de la solution TCEP/MPAA obtenue est ajusté à pH= 7,2.
Les peptides 15a ou 15b ou **15c** et 6 (1,5 eq) sont dissous séparément dans la solution précédente de TCEP/MPAA à une concentration de 3 mmol/L. Le milieu réactionnel est agité 24 h à 37°C. La cinétique de formation du produit de ligation **16** est suivie en HPLC.

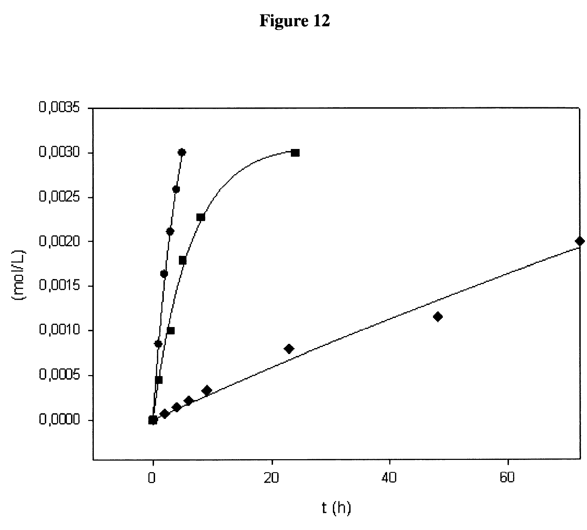

La figure 12 permet la comparaison de la cinétique de réaction de formation du peptide **16** selon la méthode de ligation SeEA, SEA ou NCL (chimie des thioesters).

Dans la figure 12, la courbe symbolisée par • correspond à la formation du peptide **16** à partir du peptide **15b,** la courbe symbolisée par ■ correspond à la formation du peptide **16** à partir du peptide **15a** et la courbe symbolisée par ◆ correspond à la formation du peptide **16** à partir du peptide **15c.**

La figure 12 montre une vitesse de réaction de ligation SeEA (courbe symbolisée par •) supérieure à celle de la méthode NCL (courbe symbolisée par ■), elle-même bien supérieure à celle de la méthode SEA (courbe symbolisée par ◆).

### Exemple 5 : Formation de segments SeEA in situ par échange et ligation simultanée-Catalyse de la réaction de ligation

### 11-Synthèse du peptide 10 : H-ILKEPVHGY-SEA

La méthode de synthèse de ce peptide est décrite dans le document Ollivier, N.; Dheur, J.; Mhidia, R.; Blanpain, A.; Melnyk, O. Organic letters 2010, 12, 5238-41.

### a- Cinétique de ligation du peptide 10 H-ILKEPVHGY-SEA avec le peptide 6 H-CILKEPVHGA-NH₂ en présence du linker 2 Se₃EA pour conduire au peptide 11, SEQ ID NO : 4 H-ILKEPVHGYCILKEPVHGA-NH₂

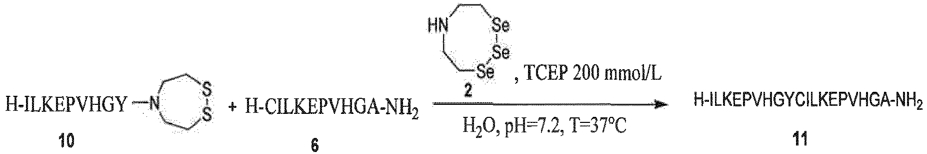

La ligation SEA du peptide **10** avec le peptide **6** est réalisée dans une boite à gant (concentration en dioxygène inférieure à 50 ppm) sous atmosphère d'azote. Une solution de TCEP à 200 mmol/L est préparée dans un tampon phosphate 0,1 M à pH 7,2.

Les peptides **10** (1 mg, 0,6 µmol) et **6** (1,4 mg, 1 µmol, 1,5 eq) ainsi que le linker 2 Se₃EA (5,8 mg, 0,02 mmol, 28 eq) sont dissous dans la solution précédente de TCEP (94 µL). Le milieu réactionnel est agité 24 h à 37°C. La cinétique de formation du produit de ligation **11** est suivie en HPLC.

### b- Comparaison de la cinétique de ligation du peptide 10 H-ILKEPVHGY-SEA avec le peptide 6 H-CILKEPVHGA-NH₂ en présence de MPAA

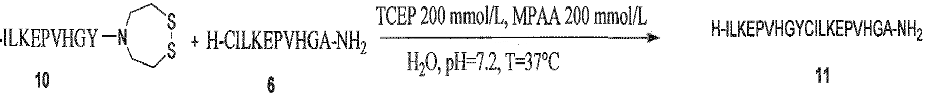

La ligation SEA du peptide **10** avec le peptide **6** est réalisée dans une boite à gant (concentration en dioxygène inférieure à 50 ppm) sous atmosphère d'azote. Une solution de TCEP à 200 mmol/L est préparée dans un tampon phosphate 0,1M à pH 7,2. L'acide 3-mercaptophénylacétique (MPAA 33 mg, 0,2 mmol) est alors additionné. Le pH de la solution TCEP/MPAA obtenue est ajusté à pH= 7,2.

Les peptides **10** (1 mg, 5 µmol) et **6** (10,6 mg, 7,6 µmol, 1,5 eq) sont dissous dans la solution précédente de TCEP/MPAA (94 µL). Le milieu réactionnel est agité 24 h à 37°C. La cinétique de formation du produit de ligation **11** est suivie en HPLC.

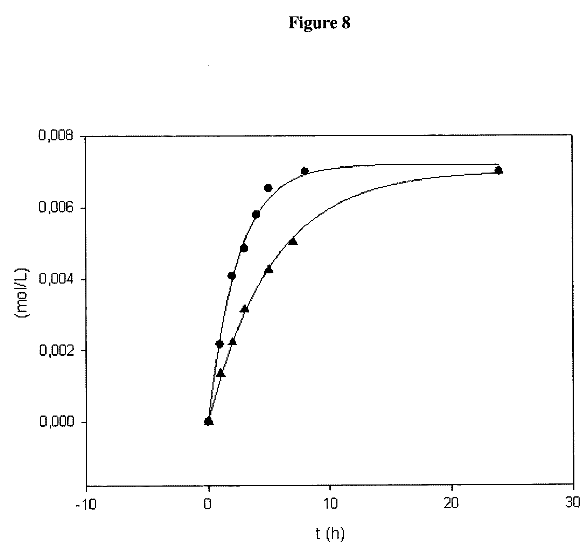

La figure 8 permet la comparaison de la cinétique de réaction de formation du peptide **11** en présence ou non du composé **2** selon l'invention.

Dans la figure 8, la courbe symbolisée par • correspond à la ligation selon l'invention du peptide **10** en présence du composé sélénié **2** et la courbe symbolisée par ▲ correspond à la ligation selon l'art antérieur du peptide **10** sans composé sélénié mais en présence de MPAA.

La figure 8 montre une vitesse de réaction plus élevée lorsque le composé **2** est présent.

### Exemple 6 : Ligation séquentielle en one-pot de trois segments à l'aide des ligations SeEA et SEA

### Synthèse du peptide 12 : H-C(StBu)HHLEPGG-SEA

La méthode de synthèse de ce peptide est décrite dans le document Ollivier, N.; Dheur, J.; Mhidia, R.; Blanpain, A.; Melnyk, O. Organic letters 2010, 12, 5238-41.

### Synthèse du peptide 14, SEQ ID NO: 5 Ac-GFGQGFGG-CHHLEPGG-CILKEPIIHGA-NH₂ par ligation des segments 4 Ac-GFGQGFGG-SeEA , 12 H-C(StBu)HHLEPGG-SEA et 6 H-CILKEPVHGA-NH₂

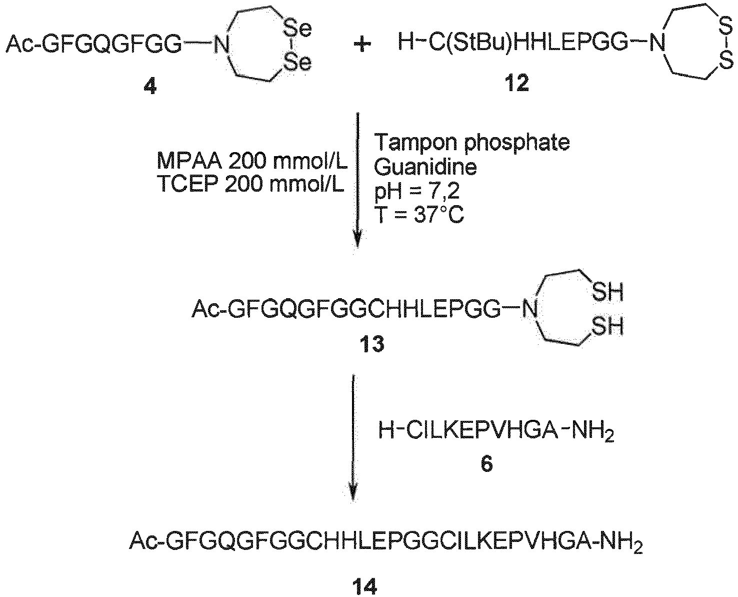

### - Etape 1

Le chlorhydrate de guanidine (Gdn,HCl, 573,18 mg, 6 mmol) est dissous dans du tampon phosphate 0,1 M pH=7,3 (1 mL final, 6M).

L'acide 4-mercaptophenylacetique (MPAA, 33,83 mg, 0,2 mmol) et le chlorhydrate de *tris*(2-carboxyethyl)phosphine (TCEP,HCl, 57,37 mg, 0,2 mmol) sont dissous dans cette solution (1 mL). 6M NaOH est ajouté pour ajuster le pH de la solution à 7,37.

Les peptides **4** Ac-GFGQGFGG-SeEA (5,33 mg, 5,4 µmol) et **12** H-C(StBu)HHLEPGG-SEA (7,59 mg, 5,4 µmol) sont dissous ensemble dans la solution précédente (780 µL).Le milieu réactionnel est placé dans un bain thermostaté à 37°C sous atmosphère inerte.

La ligation est suivie par RP-HPLC sur une colonne C18 Xbridge BEH (4,6 x 250 mm, 300 Ǻ, 5 µm) (215 nm, 1 mL/min, 30°C, tampon A eau contenant 0,1% TFA, tampon B CH₃CN/eau 4/1 contenant 0.1% TFA, 0-100% B en 30 min). Pour cela, un aliquot (2 µL) de milieu réactionnel est acidifié avec 10% TFA aqueux, extrait avec Et₂O pour éliminer MPAA avant l'analyse.

On obtient de cette façon le peptide **13** Ac-GFGQGFGG-CHHLEPGG-SEA en solution.

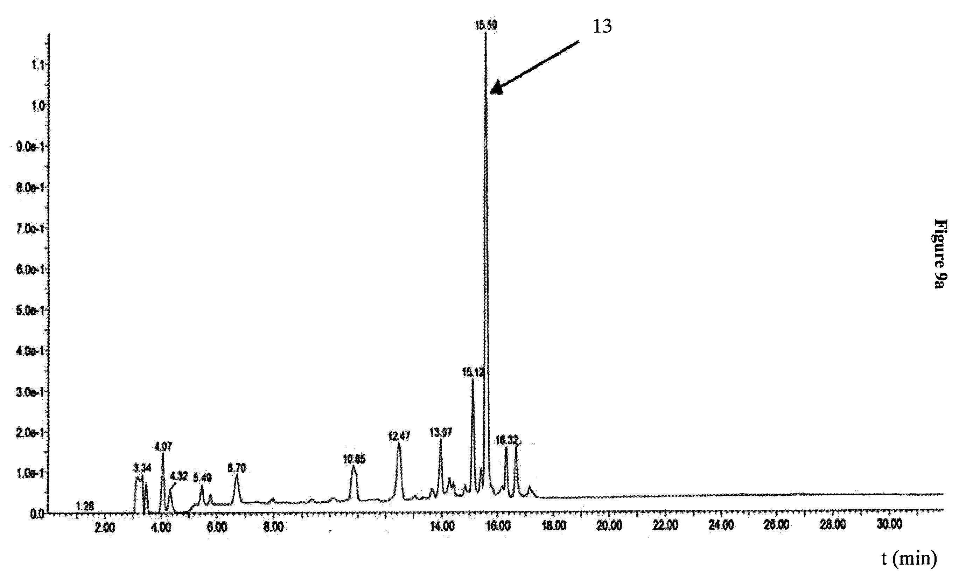

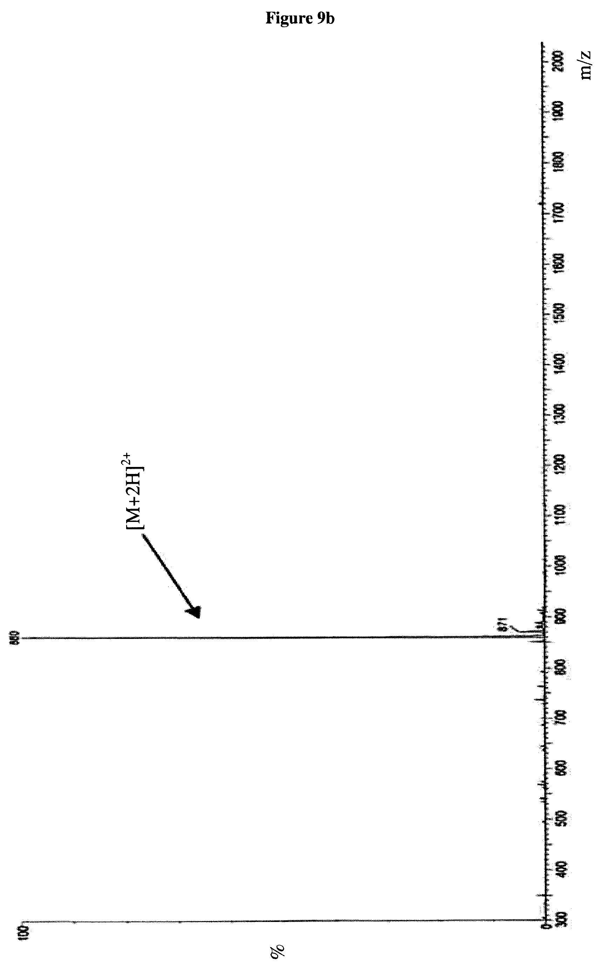

Les figures 9a et 9b représentent respectivement le chromatogramme et le spectre de masse du peptide **13.**

### - Etape 2

Après 4 h 35 de réaction, le peptide **6** H-CILKEPVHGA-NH₂ (11,06 mg, 8,1 µmol) est ajouté solide au milieu réactionnel. La réaction est placée à 37°C sous atmosphère inerte.

La ligation est suivie par RP-HPLC sur une colonne C18 Xbridge BEH (4,6 x 250 mm, 300 Ǻ, 5 µm) (215 nm, 1 mL/min, 30°C, tampon A eau contenant 0,1% TFA, tampon B CH₃CN/eau 4/1 contenant 0,1% TFA, 0-100% B en 30 min). Pour cela, un aliquot (2 µL) de milieu réactionnel est acidifié avec 10% TFA aqueux, extrait avec Et₂O pour éliminer MPAA avant l'analyse.

Lorsque la réaction est complète, le milieu réactionnel est dilué avec de l'eau (4 mL) et du TFA 10% aqueux (1 mL) est additionné. Après 4 extractions avec Et₂O (4 x 4 mL), la phase aqueuse est dégazée pendant 2 minutes par bullage d'argon. Après purification par RP-HPLC sur une colonne C18 nucléosil 120 Ǻ, 5 µm (215 nm, 6 mL/min, tampon A eau contenant 0,05% TFA, tampon B CH3CN/water 4/1 contenant 0,05% TFA, 0-10% B en 5 min, puis 10-100% B en 150 min), on obtient 6 mg (36%) de peptide **14** Ac-GFGQGFGG-CHHLEPGG-CILKEPVHGA-NH₂.

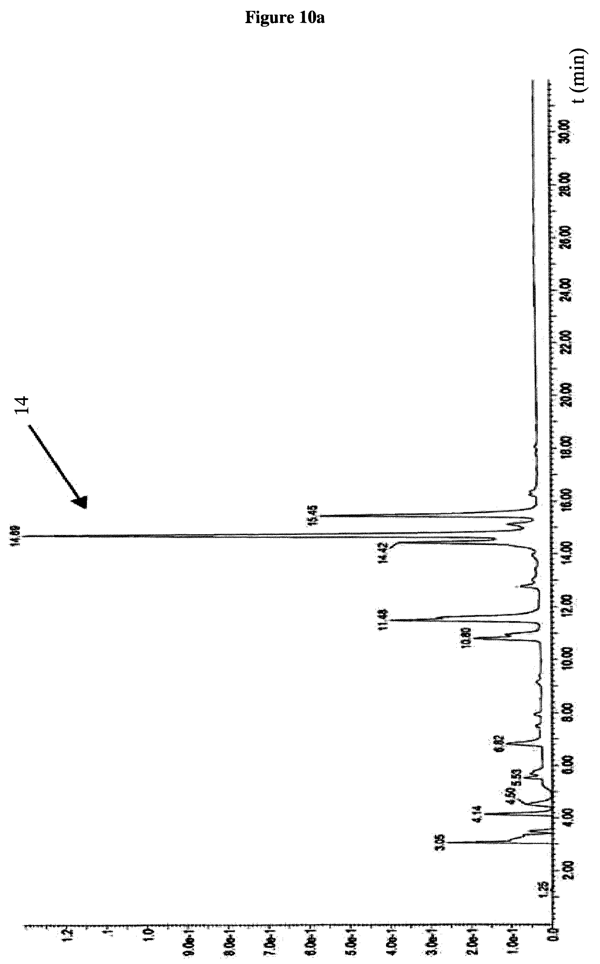

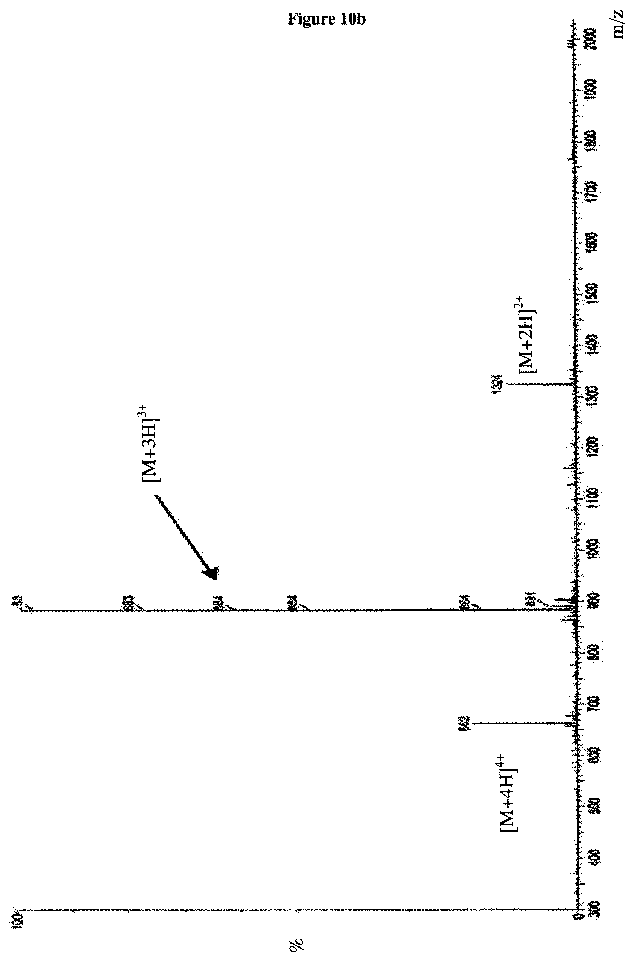

Les figures 10a et 10b représentent respectivement le chromatogramme et le spectre de masse du composé **14** avant purification.

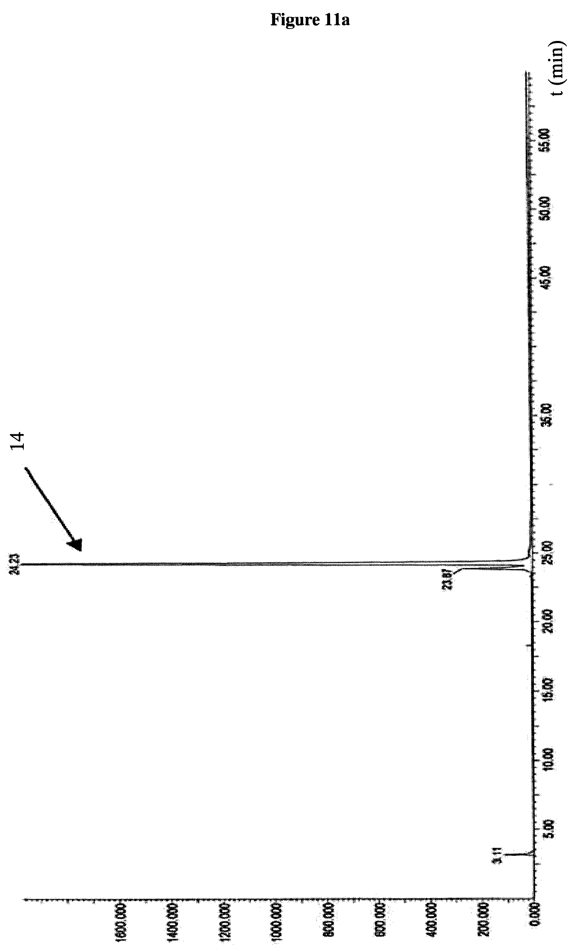

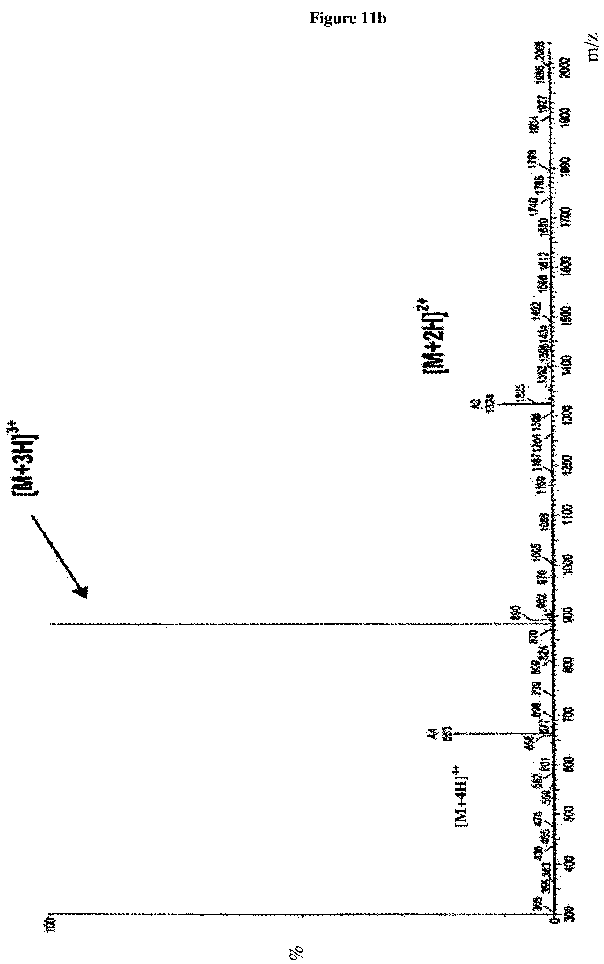

Les figures 11a et 11b représentent respectivement le chromatogramme et le spectre de masse du composé **14** après purification.

## Revendications

1. Peptide fonctionnalisé choisi parmi :
a) le peptide de formule (I) : X₁-N(CH₂CH₂SeH)₂ ; ou
b) le peptide de formule (I') : 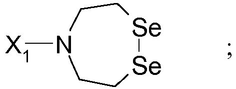 dans lesquels X₁ représente un fragment peptidique et le groupement -N(CH₂CH₂SeH)₂ ou 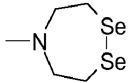 forme une liaison amide avec la terminaison C=O du résidu d'acide aminé du fragment peptidique X₁ qui est en position C-terminale.

2. Peptide fonctionnalisé selon la revendication 1, dans lequel X₁ comprend de 2 à 300 résidus d'acides aminés, de préférence de 5 à 100 résidus d'acides aminés, de manière plus particulièrement préférée de 8 à 50 résidus d'acides aminés.

3. Procédé d'obtention d'un peptide fonctionnalisé selon la revendication 1 ou selon la revendication 2, comprenant les étapes a1 et b1 suivantes :
a1) synthèse peptidique pour fournir un polypeptide de formule (II) :
(II) X₁-OH
comportant de préférence des groupements protecteurs sur ses fonctions amines et acides carboxyliques, à l'exception de sa fonction acide carboxylique C-terminale,
b1) fonctionnalisation du polypeptide obtenu à l'étape a) comprenant :
- la réaction du polypeptide de formule (II) avec un composé amine choisi parmi :
(IIIa) NH(CH₂-CH₂-Se-G₁)₂ 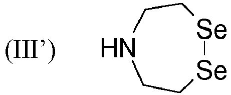
dans laquelle G₁ représente un groupement protecteur du sélénium ou un atome d'hydrogène,
en phase liquide, pour former le polypeptide de formule (Ia) X₁-N(CH₂CH₂-Se-G₁)₂, ou (I') ;
- éventuellement la déprotection du polypeptide de formule (Ia) pour conduire au composé de formule (I), ou les étapes a2 et b2 suivantes
a2) l'utilisation
- du composé de formule (IV) ou (IV") :
(IV) X₁ - N(CH₂CH₂-S-H)₂ ;
ou 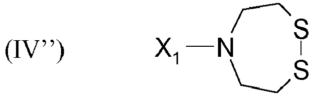
- d'un peptide fonctionnalisé par un groupement porteur d'une fonction thioester (IV') = X₁ -SR et R représente un groupement alkyle ou aryle éventuellement substitué, b2) la réaction entre le composé de formule (IV) ou (IV") ou le composé de formule (IV') avec le composé amine de formule (III') ou (III") : 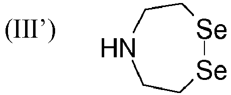 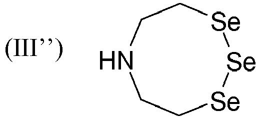 en phase liquide, pour former le peptide de formule (I) ou (I').

4. Composé susceptible d'être utilisé dans le procédé selon la revendication 3 choisi parmi :
a) le composé de formule (III') : 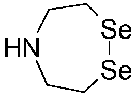
b) le composé de formule (III") :

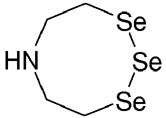

5. Procédé de fabrication des composés de formules (III') et (III") selon la revendication 4, comprenant la réaction suivante de traitement par un hydrure métallique NaBH₄, LiBH₄, ou LiAlH₄: 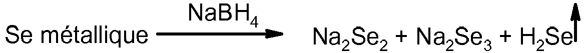 suivie de la réaction :

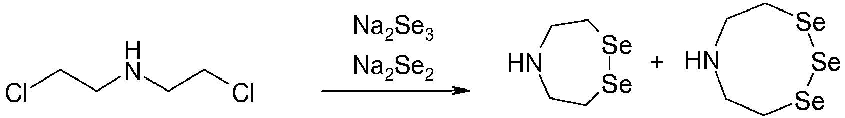

6. Procédé d'obtention d'un peptide selon la revendication 1 ou 2, comprenant les étapes suivantes :
a) Fourniture d'une résine polymère fonctionnalisée présentant la structure (V) : 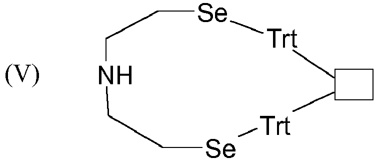 Ou la structure (V') : 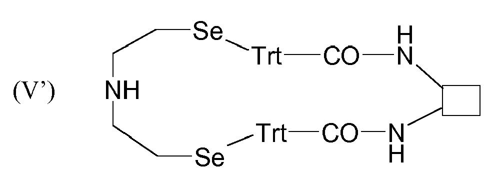 dans lesquelles,
□ représente un support solide,
Trt représente un groupement triphénylméthyle éventuellement substitué par un ou plusieurs substituants, notamment choisi parmi les substituants chlore, méthoxy, méthyle, fluor et cyano.
b) Greffage d'un acide aminé sur la résine polymère fonctionnalisée obtenue à l'étape a) pour conduire au composé de structure (VI) : 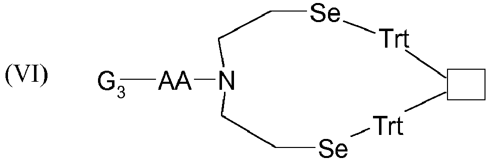 Ou de structure (VI') : 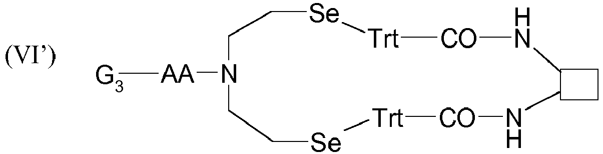 dans lesquelles,
□ et Trt ont la même signification que dans le composé (V) ou (V'), AA représente un résidu d'acide aminé comportant éventuellement un ou plusieurs groupements protecteurs ;
G₃ représente un atome d'hydrogène ou un groupement protecteur de la fonction amine N-terminale de AA,
c) Synthèse peptidique à partir de l'acide aminé AA pour conduire au composé (VII) ou (VII') : 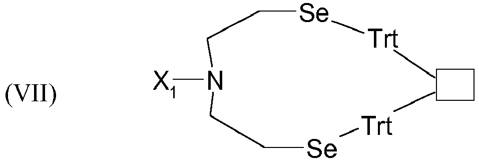 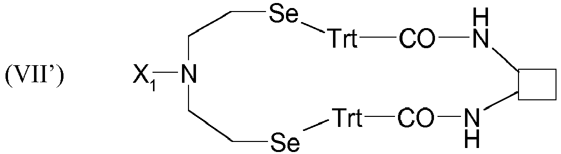 dans lesquelles,
□ représente un support solide,
Trt représente un groupement triphénylméthyle éventuellement substitué par un ou plusieurs substituants, notamment choisi parmi les substituants chlore, méthoxy, méthyle, fluor et cyano,
X₁ représente un fragment peptidique,
d) Coupure de la liaison entre Trt et Se pour conduire au composé (I) ou (I').

7. Procédé selon la revendication 6, dans lequel le greffage d'un acide aminé sur la résine polymère comprend la mise en présence de la résine fonctionnalisée (V) ou (V') avec un halogénure d'acide aminé ou avec un acide aminé et un agent d'activation, choisi de préférence parmi l'HATU, le PyBOP, le BOP, le PyBROP, de manière plus particulièrement préférée l'HATU.

8. Résine polymère fonctionnalisée susceptible d'être utilisée dans le procédé selon la revendication 6 ou 7, présentant la structure (V) : 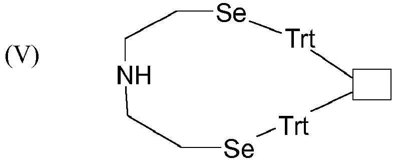 Ou la structure (V') : 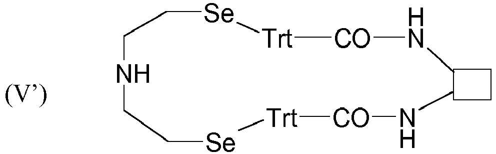 dans lesquelles,
□ représente un support solide,
Trt représente un groupement triphénylméthyle éventuellement substitué par un ou plusieurs substituants, notamment choisi parmi les substituants chlore, méthoxy, méthyle, fluor et cyano.

9. Résine polymère comprenant un fragment peptidique répondant à la formule (VII) ou (VII') : 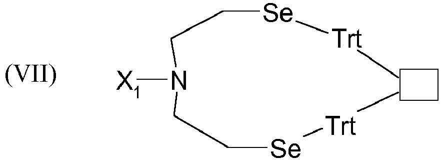 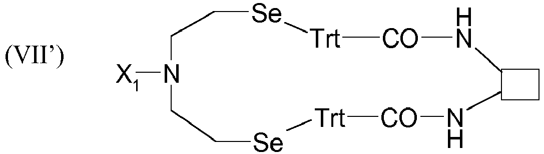 dans lesquelles,
□ représente un support solide,
Trt représente un groupement triphénylméthyle éventuellement substitué par un ou plusieurs substituants, notamment choisi parmi les substituants chlore, méthoxy, méthyle, fluor et cyano,
X₁ représente un fragment peptidique.

10. Résine polymère selon la revendication 8 ou 9, dans laquelle le support solide □ est choisi parmi un polystyrène, un polyacrylamide, un polyéthylèneglycol, une cellulose, un polyéthylène, un polyester, un latex, un polyamide, un polydiméthylacrylamide, un copolymère polyéthylèneglycol-polystyrène, un copolymère polyéthylèneglycol-polyacrylamide et leurs dérivés.

11. Procédé de fabrication de la résine polymère selon l'une des revendications 8 à 10, comprenant :
a) la fourniture d'un support solide fonctionnalisé par des groupements -Trt ou -NH-CO-Trt ;
b) réaction de la fonction Trt du support solide avec le composé amine de formule (III) : NH(CH₂-CH₂-Se-H)₂
Trt ayant la même définition que dans les revendications 8 à 10.

12. Procédé de fabrication d'un polypeptide de formule (VIII) :
(VIII) X₁-C₁-X₂-C₂-...-Cᵢ₋₁-Xᵢ-...-Cₙ₋₁-Xₙ
dans lequel,
Xᵢ, X₂,..., Xᵢ,..., Xₙ sont des fragments peptidiques,
C₁, C₂, ..., Cᵢ₋₁, ..., Cₙ₋₁ sont des résidus d'acides aminés portant une fonction thiol ou sélénol,
n est un entier supérieur ou égal à 2,
i est un nombre entier quelconque allant de 1 à n,
ce procédé comprenant une ou plusieurs étapes de réaction entre un composé de formule (IXᵢ) :
(IXᵢ) X'ᵢ - N(CH₂CH₂-Se-H)₂
dans laquelle,
X'₁ représente Xᵢ
X'ᵢ représente Cᵢ₋₁-Xᵢ pour i > 1
et un composé de formule (Xᵢ₊₁) :
(Xᵢ₊₁) Cᵢ-Xᵢ₊₁-...-Cₙ₋₁-Xₙ
pour former un composé de formule (Xᵢ) :
(Xᵢ) X'ᵢ-Cᵢ-Xᵢ₊₁-...-Cₙ₋₁-Xₙ

13. Procédé selon la revendication 12, dans lequel le composé (IXᵢ) est formé à partir du composé (IX'ᵢ) : 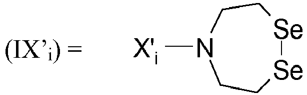 par la mise en présence d'au moins un composé réducteur des liaisons disélénium.

14. Procédé selon la revendication 13, dans lequel le composé (IX'ᵢ) est obtenu par réaction entre un composé de formule (XIᵢ), (XI'ᵢ) ou (XI"ᵢ) :
(XI_{¡}) X'ᵢ - S - R₁ 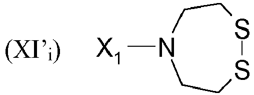
(XI"ᵢ) X'ᵢ - N(CH₂-CH₂-S-H)₂
dans lesquelles X'ᵢ est un fragment peptidique de la forme Cᵢ₋₁-Xᵢ; pour i > 1 et X'ᵢ représente Xᵢ, R₁ représente un groupement alkyle ou un groupement aryle éventuellement substitué,
et un composé de formule (III') ou de formule (III") selon la revendication 4.

15. Procédé selon la revendication 13 ou 14, pour la fabrication d'un polypeptide représenté par la formule (XII) :
(XII) X₁- C₁- X₂- C₂- X₃
comprenant les étapes suivantes :
a) réaction entre un composé de formule (XIII) ou (XIII') :
(XIII) R₂ - X₁ - N(CH₂-CH₂-Se-H)₂ 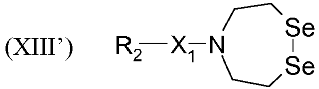
et un composé de formule (XIV) ou (XIV') :
(XIV) H - C₁ - X₂ - N(CH₂-CH₂-S-H)₂ 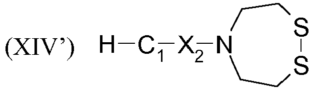
pour former le composé de formule (XV) ou (XV') :
(XV) R₂ - X₁ - C₁ - X₂ - N(CH₂-CH₂-S-H)₂ 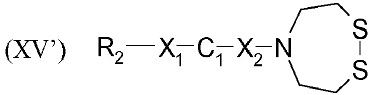
b) réaction entre un composé de formule (XV) ou (XV') et un polypeptide de formule (XVI) :
(XVI) H - C₂ - X₃
pour former le composé de formule (XII),
dans lesquelles, R₂ représente H ou un groupement protecteur de la fonction N-terminale de X₁ ; X₁, X₂, X₃ représentent, indépendamment les uns des autres, des fragments peptidiques ; C₁ et C₂ représentent, indépendamment l'un de l'autre, des résidus d'acides aminés porteurs d'une fonction thiol ou sélénol.

16. Procédé de fabrication d'un polypeptide cyclique de formule (XVII) : 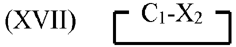 X₂ représentant un fragment peptidique, et C₁ représentant un résidu d'acide aminé comportant une fonction thiol ou sélénol,
ledit procédé comprenant au moins une étape de réaction de ligation d'un polypeptide de formule (XVIII) :
(XVIII) H - C₁ - X₂ - N(CH₂-CH₂-Se-H)₂
avec lui-même.

17. Procédé selon la revendication 16, dans lequel la réaction de ligation est effectuée en mettant en contact un polypeptide de formule (XVIII') : 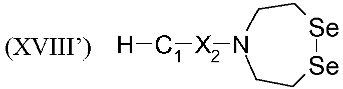 avec au moins un composé réducteur des liaisons disélénium.

18. Kit de synthèse de polypeptides comprenant un ou plusieurs peptides de formules (IXᵢ) ou (IX'ᵢ) susceptibles d'être utilisés dans le procédé selon la revendication 12 ou 13, ou au moins un composé selon la revendication 4.

## Patentansprüche

1. Funktionalisiertes Peptid, ausgewählt aus:
a) dem Peptid der Formel (I): X₁-N(CH₂CH₂-SeH)₂; oder
b) dem Peptid der Formel (I'): 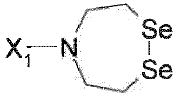 wobei X₁ für ein Peptidfragment steht und die Gruppe - N(CH₂CH₂-SeH)₂ oder 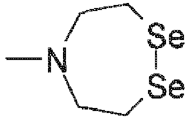 eine Amidbindung mit der Endung C=O des Aminosäurerests des Peptidfragments X₁ bildet, das sich an C-terminaler Position befindet.

2. Funktionalisiertes Peptid nach Anspruch 1, wobei Xᵢ 2 bis 300 Aminosäurereste, bevorzugt 5 bis 100 Aminosäurereste, noch stärker bevorzugt 8 bis 50 Aminosäurereste umfasst.

3. Verfahren zum Erhalt eines funktionalisierten Peptids nach Anspruch 1 oder nach Anspruch 2, umfassend die folgenden Schritte a1 und b1:
a1) Peptidsynthese, um ein Polypeptid der Formel (II) :
(II) X₁-OH
bereitzustellen, umfassend bevorzugt Schutzgruppen auf seinen Aminfunktionen und Carbonsäuren, mit Ausnahme seiner C-terminalen Carbonsäurefunktion,
b1) Funktionalisierung des in Schritt a) erhaltenen Polypeptids, umfassend:
- die Umsetzung des Polypeptids der Formel (II) mit einer Aminverbindung, ausgewählt aus:
(IIIa) NH(CH₂₋CH₂-Se-G₁)₂ 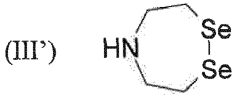
wobei G₁ für eine Selenschutzgruppe oder ein Wasserstoffatom steht,
in flüssiger Phase, um das Polypeptid der Formel (Ia)
X₁-N(CH₂CH₂-Se-G₁)₂ oder (I') zu bilden;
- gegebenenfalls die Entschützung des Polypeptids der Formel (Ia), um zur Verbindung der Formel (I) zu gelangen, oder die folgenden Schritte a2 und b2:
a2) die Verwendung
- der Verbindung der Formel (IV) oder (IV") :
(IV) X₁-N(CH₂CH₂-S-H)₂;
oder 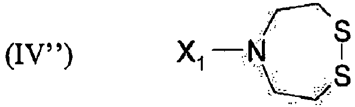
- eines Peptids, das mit einer Gruppe funktionalisiert wurde, die eine Thioesterfunktion trägt (IV') = X₁-SR und R für eine gegebenenfalls substituierte Alkyl-oder Arylgruppe steht,
b2) die Umsetzung der Verbindung der Formel (IV) oder (IV'') oder der Verbindung der Formel (IV') und der Aminverbindung der Formel (111') oder (III") : 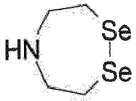 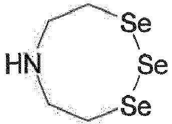 in flüssiger Phase, um das Peptid der Formel (I) oder (I') zu bilden.

4. Verbindung, die zur Verwendung in dem Verfahren nach Anspruch 3 geeignet ist, ausgewählt aus:
a) der Verbindung der Formel (III') : 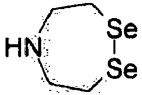
b) der Verbindung der Formel (III") .

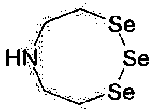

5. Verfahren zur Herstellung der Verbindungen der Formeln (III') und (111") nach Anspruch 4, umfassend die folgende Umsetzung zur Behandlung mit einem Metallhydrid NaBH₄, LiBH₄ oder LiAlH₄: 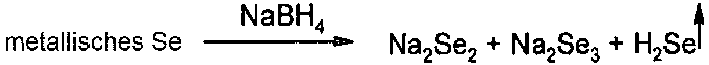 gefolgt von der Umsetzung:

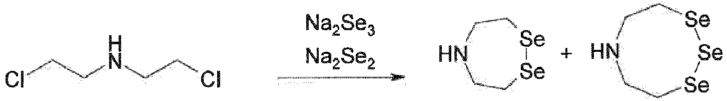

6. Verfahren zum Erhalt eines Peptids nach Anspruch 1 oder 2, umfassend die folgenden Schritte:
a) Bereitstellen eines funktionalisierten Polymerharzes, das die Struktur (V): 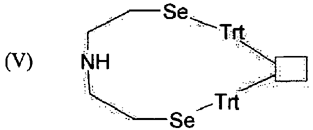 oder die Struktur (V') aufweist: 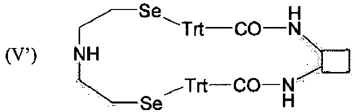 wobei
□ für einen festen Träger steht,
Trt für eine Triphenylmethylgruppe steht, die gegebenenfalls mit einem oder mehreren Substituenten substituiert ist, insbesondere ausgewählt aus den Substituenten Chlor, Methoxy, Methyl, Fluor und Cyano.
b) Pfropfen einer Aminosäure auf das in Schritt a) erhaltene funktionalisierte Polymerharz, um zur Verbindung mit der Struktur (VI): 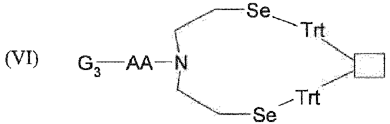 oder der Struktur (VI') 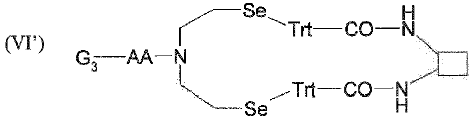
zu gelangen, wobei
□ und Trt die gleiche Bedeutung haben wie in Verbindung (V) oder (V),
AA für einen Aminosäurerest steht, der gegebenenfalls eine oder mehrere Schutzgruppen umfasst:
G₃ für ein Wasserstoffatom oder eine Schutzgruppe der N-terminalen Aminfunktion von AA steht,
c) Peptidsynthese ausgehend von der Aminosäure AA, um zu Verbindung (VII) oder (VII'): 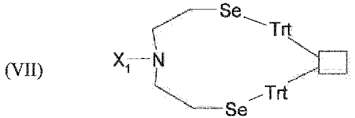 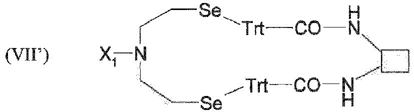
zu gelangen, wobei
□ für einen festen Träger steht,
Trt für eine Triphenylmethylgruppe steht, die gegebenenfalls mit einem oder mehreren Substituenten substituiert ist, insbesondere ausgewählt aus den Substituenten Chlor, Methoxy, Methyl, Fluor und Cyano,
X₁ für ein Peptidfragment steht,
d) Spalten der Verbindung zwischen Trt und Se, um zur Verbindung (I) oder (I') zu gelangen.

7. Verfahren nach Anspruch 6, wobei das Pfropfen einer Aminosäure auf das Polymerharz das Inkontaktbringen des funktionalisierten Harzes (V) oder (V') mit einem Aminosäurenhalogenid oder mit einer Aminosäure und einem Aktivierungsmittel umfasst, bevorzugt ausgewählt aus HATU, PyBOP, BOP, PyBroP, ganz besonders bevorzugt aus HATU.

8. Funktionalisiertes Polymerharz, das für die Verwendung in dem Verfahren nach Anspruch 6 oder 7 geeignet ist, das die Struktur (V): 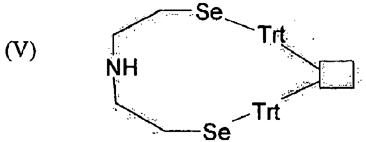 oder die Struktur (V') aufweist: 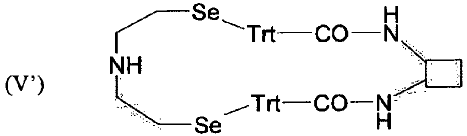 wobei
□ für einen festen Träger steht,
Trt für eine Triphenylmethylgruppe steht, die gegebenenfalls mit einem oder mehreren Substituenten substituiert ist, insbesondere ausgewählt aus den Substituenten Chlor, Methoxy, Methyl, Fluor und Cyano.

9. Polymerharz, umfassend ein Peptidfragment gemäß der Formel (VII) oder (VII'): 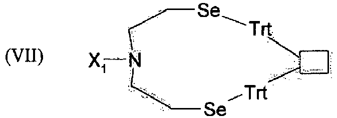 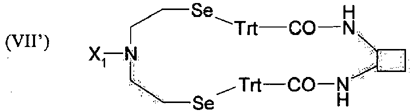 wobei
□ für einen festen Träger steht,
Trt für eine Triphenylmethylgruppe steht, die gegebenenfalls mit einem oder mehreren Substituenten substituiert ist, insbesondere ausgewählt aus den Substituenten Chlor, Methoxy, Methyl, Fluor und Cyano, X₁ für ein Peptidfragment steht.

10. Polymerharz nach Anspruch 8 oder 9, wobei der feste Träger □ ausgewählt ist aus einem Polystyrol, einem Polyacrylamid, einem Polyethylenglycol, einer Cellulose, einem Polyethylen, einem Polyester, einem Latex, einem Polyamid, einem Polydimethylacrylamid, einem Polyethylenglycol-Polystyrol-Copolymer, einem Polyethylenglycol-Polyacrylamid-Copolymer und deren Derivaten.

11. Verfahren zur Herstellung des Polymerharzes nach einem der Ansprüche 8 bis 10, umfassend:
a) das Bereitstellen eines festen Trägers, der mit den Gruppen -Trt oder -NH-CO-Trt funktionalisiert wurde;
b) Umsetzen der Funktion Trt des festen Trägers mit der Aminverbindung der Formel (III): NH(CH₂-CH₂-Se-H)₂
wobei Trt die gleiche Definition wie in den Ansprüchen 8 bis 10 aufweist.

12. Verfahren zur Herstellung eines Polypeptids der Formel (VIII):
(VIII) X₁-C₁-X₂-C₂-... -Cᵢ₋₁₋Xᵢ₋ ... -Cₙ₋₁, -Xₙ
wobei
X₁, X₂, ... , Xᵢ, ..., Xₙ Peptidfragmente sind,
C₁, C₂, ..., Cᵢ₋₁, ..., Cₙ₋₁ Aminosäurereste sind, die eine Thiol- oder Selenolfunktion tragen,
n eine ganze Zahl größer oder gleich 2 ist,
i eine beliebige ganze Zahl im Bereich zwischen 1 und n ist,
wobei dieses Verfahren einen oder mehrere Schritte zur Umsetzung einer Verbindung der Formel (IXᵢ) :
(IV) X₁-N(CH₂CH₂-S-H)₂;
wobei
X'₁ für X₁ steht,
X'ᵢ für Cᵢ₋₁-Xᵢ steht, wobei gilt: i>1
und einer Verbindung der Formel (Xᵢ₊₁) :
(Xᵢ₊₁) Cᵢ-Xᵢ₊₁-...-Cₙ₋₁-Xₙ
umfasst, um eine Verbindung der Formel (Xᵢ) zu bilden:
(Xᵢ) (X'ᵢ-Cᵢ-Xᵢ₊₁-...-Cₙ₋₁-Xₙ

13. Verfahren nach Anspruch 12, wobei die Verbindung (IXᵢ) ausgehend von der Verbindung (IX'ᵢ) : 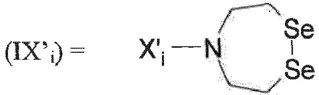 durch das Inkontaktbringen mit mindestens einer Diselenbindungen reduzierenden Verbindung gebildet wird.

14. Verfahren nach Anspruch 13, wobei die Verbindung (IX'ᵢ) durch das Umsetzen einer Verbindung der Formel (XIᵢ), (XI'ᵢ) oder (X"ᵢ) :
(XIᵢ) X'ᵢ-S-R₁ 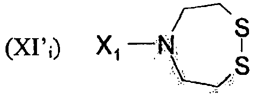
(XI"ᵢ) X'ᵢ-N(CH₂-CH₂-S-H)₂
wobei X'ᵢ ein Peptidfragment der Form Cᵢ₋₁-Xᵢ, für das gilt i>1, ist, und X'₁ für X'₁ steht, R₁ für eine gegebenenfalls substituierte Alkylgruppe oder Arylgruppe steht,
mit einer Verbindung der Formel (III') oder der der Formel (III") nach Anspruch 4 erhalten wird.

15. Verfahren nach Anspruch 13 oder 14 zur Herstellung eines Polypeptids gemäß der Formel (XII) :
(XII) X₁ - C₁ - X₂ - C₂ - X₃
das die folgenden Schritte umfasst:
a) Umsetzen einer Verbindung der Formel (XIII) oder (XIII') :
(XIII) R₂-X₁ - N(CH₂-CH₂-Se-H)₂ 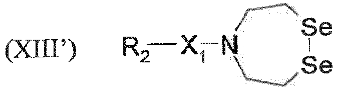
und einer Verbindung der Formel (XIV) oder (XIV') :
(XIV) H - Cl₁ - X₂ - N(CH₂-CH₂-Se-H)₂ 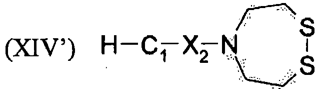
um die Verbindung der Formel (XV) oder (XV') zu bilden:
(XV) R₂ - Xᵢ - C₁ - X₂-N(CH₂-CH₂-S-H)₂ 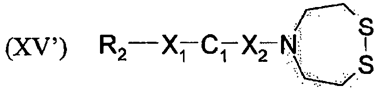
b) Umsetzen einer Verbindung der Formel (XV) oder (XV') und eines Polypeptids der Formel (XVI) :
(XVI) H - C₂ - X₃
um die Verbindung der Formel (XII) zu bilden,
wobei R₂ für H oder eine Schutzgruppe der N-terminalen Funktion von Xᵢ steht; X₁, X₂, X₃ unabhängig voneinander für Peptidfragmente stehen; C₁ und C₂ unabhängig voneinander für Aminosäurereste stehen, die eine Thiol- oder Selenolfunktion tragen.

16. Verfahren zur Herstellung eines cyclischen Polypeptids der Formel (XVII) : 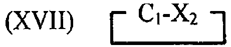
wobei X₂ für ein Peptidfragment steht und C₁ für einen Aminosäurerest steht, der eine Thiol- oder Selenolfunktion umfasst,
wobei das Verfahren mindestens einen Schritt zur Umsetzung mittels Ligation eines Polypeptids der Formel (XVIII):
(XVIII) H - C₁ - X₂ - N(CH₂-CH₂-Se-H)₂
mit sich selbst umfasst.

17. Verfahren nach Anspruch 16, wobei die Umsetzung mittels Ligation durchgeführt wird, indem ein Polypeptid der Formel (XVIII') : 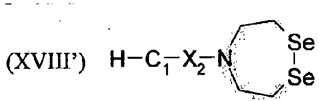 mit mindestens einer Diselenbindungen reduzierenden Verbindung in Kontakt gebracht wird.

18. Kit zur Synthese von Polypeptiden, umfassend ein oder mehrere Peptide der Formeln (IXᵢ) oder (IX'ᵢ), die für die Verwendung in dem Verfahren nach Anspruch 12 oder 13 geeignet sind, oder mindestens eine Verbindung nach Anspruch 4.

## Claims

1. Functionalized peptide selected from:
a) the peptide of formula (I): X₁-N(CH₂CH₂SeH)₂; or
b) the peptide of formula (I'): 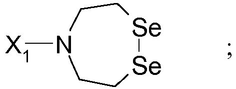 in which X₁ represents a peptide fragment and the -N(CH₂CH₂SeH)₂ group or 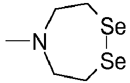 group forms an amide bond with the C=O termination of the amino acid residue of the peptide fragment X₁ which is in the C-terminal position.

2. Functionalized peptide according to claim 1, in which X₁ comprises from 2 to 300 amino acid residues, preferably from 5 to 100 amino acid residues, more particularly preferably from 8 to 50 amino acid residues.

3. Process for obtaining a functionalized peptide according to claim 1 or according to claim 2, comprising the following steps a1 and b1:
a1) peptide synthesis for providing a polypeptide of formula (II):
(II) X₁-OH
preferably comprising protective groups on its amine and carboxylic acid functions, with the exception of its C-terminal carboxylic acid function,
b1) functionalization of the polypeptide obtained in step a) comprising:
- the reaction of the polypeptide of formula (II) with an amine compound selected from:
(IIIa) NH(CH₂-CH₂-Se-G₁)₂ 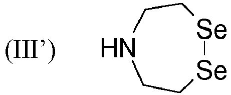
in which G₁ represents a selenium protective group or a hydrogen atom,
in liquid phase, in order to form the polypeptide of formula (Ia) X₁-N(CH₂CH₂-Se-G₁)₂, or (I');
- optionally the deprotection of the polypeptide of formula (Ia) in order to produce the compound of formula (I),
or the following steps a2 and b2
a2) the use
- of the compound of formula (IV) or (IV"):
(IV) X₁-N(CH₂CH₂-S-H)₂;
or 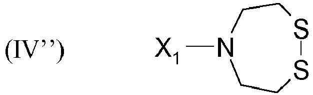
- of a peptide functionalized by a group bearing a thioester function (IV') = X1 -SR and R represents an optionally substituted aryl or alkyl group,
b2) the reaction between the compound of formula (IV) or (IV") or the compound of formula (IV') with the amine compound of formula (III') or (III"): 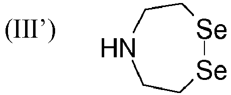 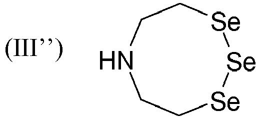 in liquid phase, in order to form the peptide of formula (I) or (I').

4. Compound capable of being used in the process according to claim 3 selected from:
a) the compound of formula (III'): 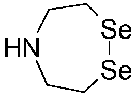
b) the compound of formula (III"):

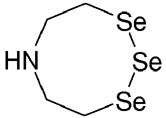

5. Process for producing the compounds of formulae (III') and (III") according to claim 4, comprising the following treatment reaction with a metal hydride NaBH, LiBH₄, or LiAlH₄: 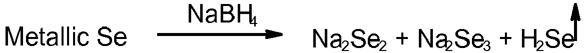 followed by the reaction:

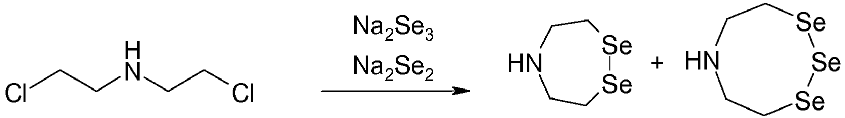

6. Process for obtaining a peptide according to claim 1 or 2, comprising the following steps:
a) Provision of a functionalized polymer resin with the structure (V): 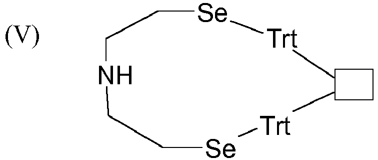 Or the structure (V'): 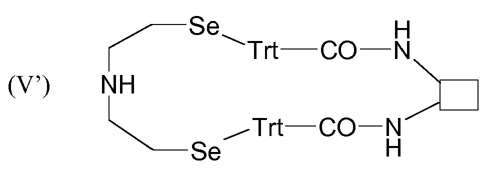 in which,
□ represents a solid support,
Trt represents a triphenylmethyl group optionally substituted by one or more substituents, in particular selected from the substituents chlorine, methoxy, methyl, fluorine and cyano.
b) Grafting of an amino acid onto the functionalized polymer resin obtained in step a) in order to produce the compound of structure (VI): 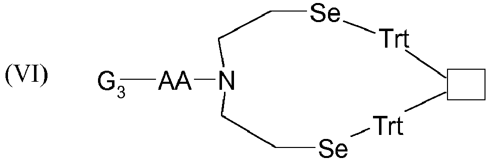 Or of structure (VI'): 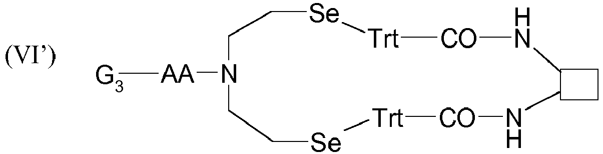 in which,
□ and Trt have the same meaning as in compound (V) or (V'),
AA represents an amino acid residue optionally comprising one or more protective groups;
G₃ represents a hydrogen atom or a protective group of the N-terminal amine function of AA,
c) Peptide synthesis starting from the amino acid AA in order to produce compound (VII) or (VII'): 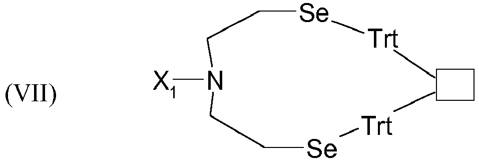 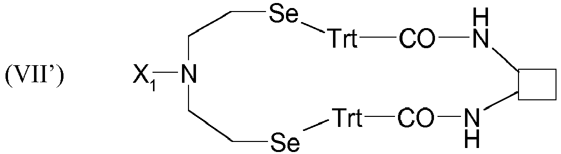 in which,
□ represents a solid support,
Trt represents a triphenylmethyl group optionally substituted by one or more substituents, in particular selected from the substituents chlorine, methoxy, methyl, fluorine and cyano,
X₁ represents a peptide fragment,
d) Cleavage of the bond between Trt and Se in order to produce compound (I) or (I').

7. Process according to claim 6, in which the grafting of an amino acid onto the polymer resin comprises placing the functionalized resin (V) or (V') in contact with an amino acid halide or with an amino acid and an activation agent, preferably selected from HATU, PyBOP, BOP, PyBROP, more particularly preferably HATU.

8. Functionalized polymer resin capable of being used in the process according to claim 6 or 7, with the structure (V): 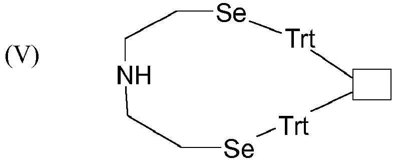 Or the structure (V'): 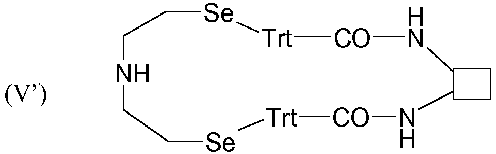 in which,
□ represents a solid support,
Trt represents a triphenylmethyl group optionally substituted by one or more substituents, in particular selected from the substituents chlorine, methoxy, methyl, fluorine and cyano.

9. Polymer resin comprising a peptide fragment corresponding to formula (VII) or (VII'): 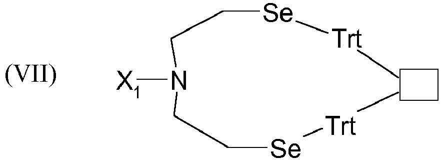 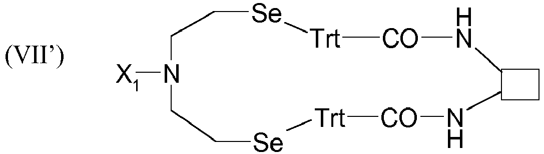 in which,
□ represents a solid support,
Trt represents a triphenylmethyl group optionally substituted by one or more substituents, in particular selected from the substituents chlorine, methoxy, methyl, fluorine and cyano,
X₁ represents a peptide fragment.

10. Polymer resin according to claim 8 or 9, in which the solid support □ is selected from a polystyrene, a polyacrylamide, a polyethylene glycol, a cellulose, a polyethylene, a polyester, a latex, a polyamide, a polydimethylacrylamide, a polyethylene glycol-polystyrene copolymer, a polyethylene glycolpolyacrylamide copolymer and derivatives thereof.

11. Process for producing the polymer resin according to one of claims 8 to 10, comprising:
a) the provision of a functionalized solid support by -Trt or -NH-CO-Trt groups;
b) reaction of the Trt function of the solid support with the amine compound of formula (III): NH(CH₂-CH₂-Se-H)₂
Trt having the same definition as in claims 8 to 10.

12. Process for producing a polypeptide of formula (VIII):
(VIII) X₁-C₁-X₂-C₂-...-Cᵢ₋₁-Xᵢ-...-Cₙ₋₁-Xₙ
in which,
X₁, X₂,..., Xᵢ,..., Xₙ are peptide fragments,
C₁₅ C₂, ..., Cᵢ₋₁, ..., Cₙ₋₁ are amino acid residues bearing a thiol or selenol function,
n is an integer greater than or equal to 2,
i is any integer in the range from 1 to n,
this process comprising one or more steps of reaction between a compound of formula (IXᵢ):
(IXᵢ) X'ᵢ - N(CH₂CH₂-Se-H)₂
in which,
X'ᵢ represents X₁
X'ᵢ represents Cᵢ₋₁-Xᵢ for i > 1
and a compound of formula (Xᵢ₊₁):
(Xᵢ₊₁) Cᵢ-Xᵢ₊₁-...-Cₙ₋₁-Xₙ
in order to form a compound of formula (Xᵢ):
(Xᵢ) X'ᵢ-Cᵢ-Xᵢ₊₁-...-Cₙ₊₁-Xₙ

13. Process according to claim 12, in which compound (IXᵢ) is formed starting from compound (IX'ᵢ): 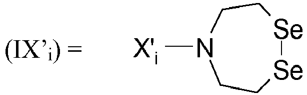 by placing in contact with at least one compound that reduces diselenium bonds.

14. Process according to claim 13, in which compound (IX'ᵢ) is obtained by reaction between a compound of formula (XIᵢ), (XI'ᵢ) or (XI"ᵢ):
(XIᵢ) X'ᵢ - S - R₁ 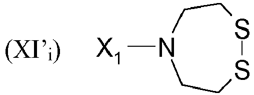
(XI"ᵢ) X'ᵢ - N(CH₂-CH₂-S-H)₂
in which X'ᵢ is a peptide fragment of the form Cᵢ₋₁-Xᵢ for i > 1 and X'₁ represents X₁, R₁ represents an optionally substituted aryl or alkyl group, and a compound of formula (III') or of formula (III") according to claim 4.

15. Process according to claim 13 or 14, for the production of a polypeptide represented by formula (XII):
(XII) X₁ - C₁- X₂ - C₂- X₃
comprising the following steps:
a) reaction between a compound of formula (XIII) or (XIII'):
(XIII) R₂ - X₁ - N(CH₂-CH₂-Se-H)₂ 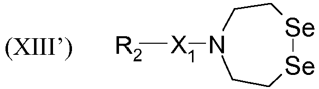
and a compound of formula (XIV) or (XIV'):
(XIV) H - C₁ - X₂ - N(CH₂-CH₂-S-H)₂ 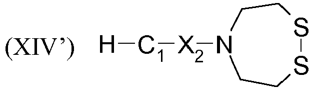
in order to form the compound of formula (XV) or (XV'):
(XV) R₂ - X₁ - C₁ - X₂ - N(CH₂-CH₂-S-H)₂ 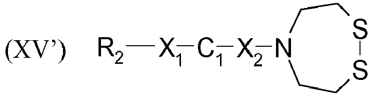
b) reaction between a compound of formula (XV) or (XV') and a polypeptide of formula (XVI):
(XVI) H -C₂ - X₃
in order to form the compound of formula (XII),
in which, R₂ represents H or a protective group of the N-terminal function of Xᵢ; X₁, X₂, X₃ represent, independently of each other, peptide fragments; C₁ and C₂ represent, independently of each other, amino acid residues bearing a thiol or selenol function.

16. Process for producing a cyclic polypeptide of formula (XVII): 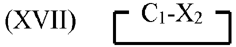 X₂ representing a peptide fragment, and C₁ representing an amino acid residue comprising a thiol or selenol function,
said process comprising at least one reaction step of ligation of a polypeptide of formula (XVIII):
(XVIII) H - C₁ - X₂ - N(CH₂-CH₂-Se-H)₂
with itself.

17. Process according to claim 16, in which the ligation reaction is carried out by placing a polypeptide of formula (XVIII'): 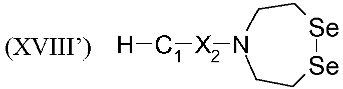 in contact with at least one compound that reduces diselenium bonds.

18. Polypeptide synthesis kit comprising one or more peptides of formulae (IXᵢ) or (IX'ᵢ) capable of being used in the process according to claim 12 or 13, or at least one compound according to claim 4.


### SEQUENCE LISTING

<110> CENTRE NATIONAL DE LA RECHERCHE SCIENTIFIQUE
   UNIVERSITE LILLE 2 DROIT ET SANTE
   INSTITUT PASTEUR DE LILLE
<120> PROCEDE DE LIGATION NATIVE
<130> 33456 cnrs
<160> 5
<170> patentIn version 3.5
<210> 1
   <211> 8
   <212> PRT
   <213> Artificial Sequence
<220>
   <223> PEPTIDE 3 ACETYLE
<400> 1
   Gly Phe Gly Gln Gly Phe Gly Gly
   1 5
<210> 2
   <211> 10
   <212> PRT
   <213> Artificial Sequence
<220>
   <223> PEPTIDE 6
<400> 2
   Cys Ile Leu Lys Glu Pro Val His Gly Ala
   1 5 10
<210> 3
   <211> 18
   <212> PRT
   <213> Artificial Sequence
<220>
   <223> PEPTIDE 7 ACETYLE
<400> 3
   Gly Phe Gly Gln Gly Phe Gly Gly Cys Ile Leu Lys Glu Pro Val His
   1 5 10 15
   Gly Ala
<210> 4
   <211> 19
   <212> PRT
   <213> Artificial Sequence
<220>
   <223> PEPTIDE 11
<400> 4
   Ile Leu Lys Glu Pro Val His Gly Tyr Cys Ile Leu Lys Glu Pro Val
   1 5 10 15
   His Gly Ala
<210> 5
   <211> 26
   <212> PRT
   <213> Artificial Sequence
<220>
   <223> PEPTIDE 14 ACETYLE
<400> 5
   Gly Phe Gly Gln Gly Phe Gly Gly Cys His His Leu Glu Pro Gly Gly
   1               5                   10                  15
   Cys Ile Leu Lys Glu Pro Val His Gly Ala
               20                  25